(19) Europäisches Patentamt
European Patent Office
Office européen des brevets

(11) EP 4 023 224 B1

(12) **EUROPEAN PATENT SPECIFICATION**

(45) Date of publication and mention of the grant of the patent:
**28.01.2026 Bulletin 2026/05**

(21) Application number: **20856251.2**

(22) Date of filing: **25.08.2020**

(51) International Patent Classification (IPC):
***A61K 31/548*** (2006.01) ***A61K 31/549*** (2006.01)
***A61P 35/00*** (2006.01)

(52) Cooperative Patent Classification (CPC):
**A61P 35/00; A61K 31/549**

(86) International application number:
**PCT/KR2020/011346**

(87) International publication number:
**WO 2021/040389 (04.03.2021 Gazette 2021/09)**

(54) **PHARMACEUTICAL COMPOSITION FOR PREVENTING OR TREATING SOLID CANCER, CONTAINING EPIDITHIODIOXOPIPERAZINE DERIVATIVE OR PHARMACEUTICALLY ACCEPTABLE SALTS THEREOF**

PHARMAZEUTISCHE ZUSAMMENSETZUNG ZUR VORBEUGUNG ODER BEHANDLUNG VON SOLIDEN TUMOREN ENTHALTEND EIN EPIDITHIODIOXOPIPERAZINDERIVAT ODER DESSEN PHARMAZEUTISCH VERTRÄGLICHE SALZE

COMPOSITION PHARMACEUTIQUE DESTINÉE À PRÉVENIR OU À TRAITER LE CANCER SOLIDE, CONTENANT UN DÉRIVÉ DE L'ÉPIDITHIODIOXOPIPÉRAZINE OU DES SELS PHARMACEUTIQUEMENT ACCEPTABLES DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2019 KR 20190105964**

(43) Date of publication of application:
**06.07.2022 Bulletin 2022/27**

(73) Proprietor: **VASTHERA Co. Ltd.**
**Seoul 03760 (KR)**

(72) Inventors:
- **KANG, Sang Won**
  **Seoul 06517 (KR)**
- **LEE, Eun Kyung**
  **Seoul 05680 (KR)**

(74) Representative: **Sagittarius IP**
**Marlow International**
**Parkway**
**Marlow SL7 1YL (GB)**

(56) References cited:
**WO-A1-02/26744**
**WO-A1-2006/066775**
**WO-A1-93/17715**
**WO-A2-2006/135949**
**CA-A1- 2 252 663**
**US-A1- 2009 029 974**
**US-A1- 2017 239 261**

- **MARCUS BAUMANN , ANDRÉ P. DIESKAU , BRAD M. LOERTSCHER , MARY C. WALTON , SANGKIL NAM , JUN XIE , DAVID HORNE , LARRY E. OVERMAN: "Tricyclic analogues of epidithiodioxopiperazine alkaloids with promising in vitro and in vivo antitumor activity", CHEMICAL SCIENCE, vol. 6, no. 8, 1 January 2015 (2015-01-01), pages 4451 - 4457, XP055786689, DOI: 10.1039/C5SC01536G**
- **WELCH, T. R. et al. Epidithiodioxopiperazines. occurrence, synthesis and biogenesis. Natural Product Reports. 2014, vol. 31, pp. 1376-1404 See abstract; page 1384; and figures 1-3. X 1-3,5-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DONG HOON KANG, DOO JAE LEE, JIRAN KIM, JOO YOUNG LEE, HYUN-WOO KIM, KIHWAN KWON, W. ROBERT TAYLOR, HANJOONG JO, SANG WON KANG: "Vascular Injury Involves the Overoxidation of Peroxiredoxin Type II and Is Recovered by the Peroxiredoxin Activity Mimetic That Induces Reendothelialization", CIRCULATION, vol. 128, no. 2, 2 July 2013 (2013-07-02), pages 834 - 844, XP055786700, DOI: 10.1161/circulationaha.113.001725**

## Description

### [Technical Field]

The present invention relates to a pharmaceutical composition for prevention or treatment of solid cancer comprising an epidithiodioxopiperazine derivative compound based on a parent structure comprising an intramolecular disulfide bridge in an epidithiodioxopiperazine ring or a pharmaceutically acceptable salt thereof as an active ingredient.

### [Background Art]

Cancer (tumor) may be defined simply as a generic term for cells that proliferate and grow faster than normal cells in the body. Cancer is also called a tumor, and tumors are divided into benign tumors, in which only normal cells grow and proliferate rapidly to increase in size and form a lump, and malignant tumors, in which abnormally transformed cells grow and/or proliferate and metastasize to other parts, and cancer commonly refers to such malignant tumors.

Such cancer may be broadly divided into solid cancer and blood cancer. Solid cancer is one that develops in a specific organ, grows, and metastasizes to other organs, such as stomach cancer or lung cancer. On the other hand, cancer which develops in the hematopoietic organ that produces blood or immune cells rather than a specific organ, is called blood cancer since the cancer cells circulate in the blood or lymph system instead of being located in a specific site.

Such solid cancer and blood cancer are treated by way of different methods as their origins, developing locations, and mechanisms of metastasis are different from each other. In the case of solid cancer, since the cancer develops in a specific organ, surgery or radiation therapy is performed when the cancer is confined to a specific organ, but chemotherapy is performed when such local treatment is difficult. However, in the case of blood cancer, since the cancer is not limited to a specific site, chemotherapy is the main treatment regardless of the degree of disease progression.

### [Disclosure]

### [Technical Problem]

The present inventors have made intensive research efforts to discover small-molecule compounds that are derived from natural products and/or synthetic small-molecule compounds that can mimic the functions of the natural product-derived small-molecule compounds for the treatment of solid cancer, and as a result, they have confirmed that natural epidithiodioxopiperazine derivatives such as gliotoxin and chetomin and synthetic small-molecule derivatives sharing a similar parent structure with the natural epidithiodioxopiperazine derivatives exhibit the activity of inhibiting the growth, proliferation, and/or metastasis of breast cancer, lung cancer, stomach cancer, skin cancer, colon cancer, prostate cancer, and pancreatic cancer, thereby completing the present invention.

### [Technical Solution]

An object of the present invention to provide a pharmaceutical composition for prevention or treatment of solid cancer comprising an epidithiodioxopiperazine derivative compound based on a parent structure comprising an intramolecular disulfide bridge in an epidithiodioxopiperazine ring or a pharmaceutically acceptable salt thereof as an active ingredient.

The invention is defined in the claims. Any subject-matter beyond the scope of the claims is not part of the invention and provided for reference or comparison purposes only.

More specifically, the present invention concerns synthetic epidithiodioxopiperazine derivatives as defined in the claims for use in the prevention or treatment of a solid cancer selected from breast cancer, lung cancer, stomach cancer, prostate cancer, or pancreatic cancer.

Any reference to a method of treatment of the human or animal body by therapy using a certain compound or composition is to be understood as a reference to the use of said compound or composition for use in said method of treatment.

### [Advantageous Effects]

The pharmaceutical composition comprising natural and synthetic small-molecular epidithiodioxopiperazine derivatives of the present invention can efficiently kill breast cancer, lung cancer, stomach cancer, skin cancer, colon cancer, prostate cancer, and pancreatic cancer cells without affecting cell viability as well as inhibit the growth of tumors or further decrease the volume of tumors in mouse models implanted with tumors.

**[Description of Drawings]**

Figure 1 (reference) is diagrams illustrating (A) the viability and (B) and (C) the proliferation inhibitory effect when breast cancer cells are treated with natural epidithiodioxopiperazine (ETP) compounds, gliotoxin, and chetomin at various concentrations.

Figure 2 (reference) is diagrams illustrating the cell cycle progression inhibitory effect of gliotoxin on triple-negative breast cancer (TNBC) cells.

Figure 3 (reference) is diagrams illustrating the migratory activity inhibitory effect of a natural epidithiodioxopiperazine compound, gliotoxin, on breast cancer cells.

Figure 4 (reference) is diagrams illustrating the migratory activity inhibitory effect of a natural epidithiodioxopiperazine compound, chetomin, on breast cancer cells.

Figure 5 is diagrams illustrating the cytotoxic effect of a synthetic epidithiodioxopiperazine derivative on various cancer cells and normal cells. (A) to (J) respectively illustrate the cell viability quantified by the Cell Titer-Glo assay system of breast cancer cells, lung cancer cells, stomach cancer cells, skin cancer cells, colon cancer cells, prostate cancer cells, pancreatic cancer cells, normal breast cells, normal fibroblasts, and normal epithelial cells after treating with the labelled ETP compound for 24 hours (n = 3). And $IC_{50}$ values are determined using a linear regression program (GraphPad Prism) and illustrated in (K).

Figure 6 is diagrams illustrating the colony formation inhibitory effect of a synthetic epidithiodioxopiperazine derivative on breast cancer cells.

Figure 7 is diagrams illustrating the colony formation inhibitory effect of a synthetic epidithiodioxopiperazine derivative on lung cancer cells.

Figure 8 is diagrams illustrating the colony formation inhibitory effect of a synthetic epidithiodioxopiperazine derivative on stomach cancer cells.

Figure 9 (reference) is diagrams illustrating the colony formation inhibitory effect of a synthetic epidithiodioxopiperazine derivative on skin cancer cells.

Figure 10 is diagrams illustrating the colony formation inhibitory effect of a synthetic epidithiodioxopiperazine derivative on colon cancer cells.

Figure 11 is diagrams illustrating the colony formation inhibitory effect of a synthetic epidithiodioxopiperazine derivative on prostate cancer cells.

Figure 12 is diagrams illustrating the colony formation inhibitory effect of a synthetic epidithiodioxopiperazine derivative on pancreatic cancer cells.

Figure 13 is diagrams illustrating the tumor growth inhibitory effect of a synthetic epidithiodioxopiperazine derivative on A549, SK-MEL-28, MKN-28 and Luc-MDA-MB-231 xenografts. 100 mL of vehicle control group (0.1% DMSO) and the labelled ETP compound (300 $\mu$g/kg) have been intraperitoneally injected every 3 days for 20 days, and xenografts have been harvested from the mice of each group after 4 weeks. (A) to (D) illustrate the tumor xenograft volumes and masses measured in A549, SK-MEL-28, MKN-28, and Luc-MDA-MB-231 xenograft mice treated with the control (black), A2 (red), A5 (green), and A10 (yellow), respectively. Data in the graphs are expressed as mean $\pm$ S.E.M (n = 4, **P < 0.05). The statistical analysis has been performed by one-way ANOVA with Tukey HSD *post hoc* test.

Figure 14 is diagrams illustrating the *in vivo* cancer cell proliferation inhibitory effect of a synthetic epidithiodioxopiperazine derivative. (A) and (C), and (B) and (D) illustrate the proliferative index and immunohistochemistry determined by Ki-67 immunostaining of MDA-MB231-derived and MKN28-derived tumors. Data in the graphs are the mean $\pm$ S.D. (n = 4, **P < 0.001) of the number of Ki-67$^+$ cells per field from immunofluorescence staining. (E) and (F) illustrate the ETP-induced apoptosis incidence determined by TUNEL staining in MDA-MB231 and MKN-28 induced tumor xenografts, respectively. (G) is a DNase-treated sample used as a positive control group for TUNEL staining. Specifically, the tissue sections have been treated with DNase I (10 U/mL) for 2 minutes.

Figure 15 is diagrams illustrating the inhibitory effect of a synthetic epidithiodioxopiperazine derivative on patient-derived lung adenocarcinoma implanted into humanized mice.

**[Best Mode]**

The present disclosure provides a pharmaceutical composition for the prevention or treatment of solid cancer comprising an epidithiodioxopiperazine derivative compound based on a parent structure comprising an intramolecular disulfide bridge in an epidithiodioxopiperazine ring represented by the following Chemical Formula 1 or a pharmaceutically acceptable salt thereof as an active ingredient:

[Chemical Formula 1]

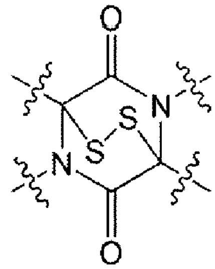

The present disclosure is based on the discovery that a series of natural or synthetic small-molecule compounds having an intramolecular disulfide bridge on the epidithiodioxopiperazine ring exert a therapeutic effect on solid cancer diseases through the intramolecular disulfide bridge contained therein. Specifically, the present inventors have confirmed that gliotoxin and chetomin, which are representative natural product-derived epidithiodioxopiperazine derivatives, and the small-molecule epidithiodioxopiperazine derivatives 5,7-dimethyl-2,3-dithia-5,7-diazabicyclo[2.2.2]octane-6,8-dione (A2), 5,7-diallyl-2,3-dithia-5,7-diazabicyclo[2.2.2]octane-6,8-dione (A5), and 5a,10a-dithio-octahydrodipyrrolo [1,2-*a*:1',2'-*d*]pyrazine-5,10-dione (A10), which are synthesized to identify the key mechanisms of action, all exert an excellent therapeutic effect on solid cancers such as breast cancer, lung cancer, stomach cancer, skin cancer, colon cancer, prostate cancer, and pancreatic cancer, and it is thus self-evident that epidithiodioxopiperazine derivatives comprising an intramolecular disulfide bridge can exert a therapeutic effect on solid cancer regardless of their sizes or the kinds of substituents. Only compounds as defined by the appended claims are part of the invention.

The epidithiodioxopiperazine derivative compound comprised in the pharmaceutical composition of the present disclosure may be a natural product-derived epidithiodioxopiperazine compound represented by the following Chemical Formula 2 or 3:

[Chemical Formula 2]

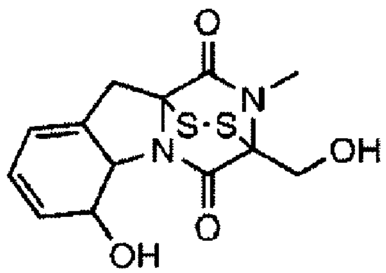

[Chemical Formula 3]

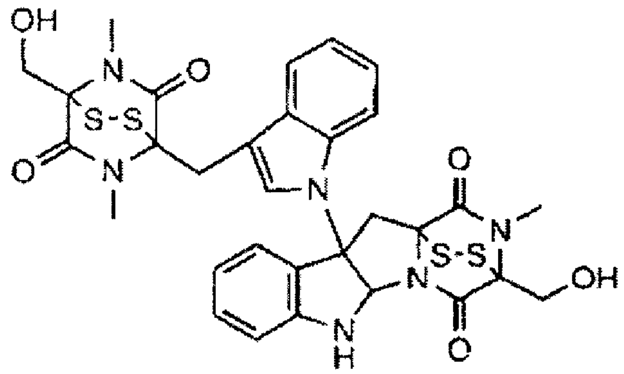

The compound represented by Chemical Formula 2 is a representative ETP compound called gliotoxin (GT), and may be isolated from fungi such as *Aspergillus fumigatus, Trichoderma virens, Penicillium* spp., or *Candida albicans* or culture thereof, metabolites thereof, or secondary metabolites thereof (Kirby and Robins, 1980, The Biosynthesis of

Mycotoxins, New York: Academic Press; Shah and Larsen, 1991, Mycopathologia, 116: 203-208).

The compound represented by Chemical Formula 3 is an ETP compound called chetomin, and may be isolated from *Chaetomium globosum* (Sekita et al., 1981, Can. J. Microbiol., 27: 766-772).

Baumann et al. (Chemical Science, vol. 6, no. 8, 2015, pages 4451-4457) disclose tricyclic analogues of epidithiodioxopiperazine alkaloids which were found to have promising *in vitro* and *in vivo* antitumor activity.

The epidithiodioxopiperazine derivative compound comprised in the pharmaceutical composition of the present invention is a synthetic small-molecule compound represented by the following [Chemical Formula 1-1]:

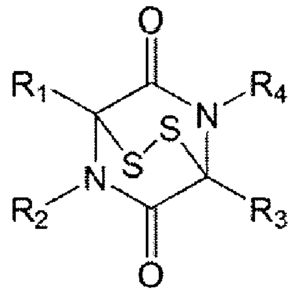

in Chemical Formula 1-1,

$R_1$ to $R_4$ are each independently hydrogen, linear or branched $C_{1-6}$ alkyl, alkenyl, alkynyl, $C_{1-6}$ alkoxy-aryl-$C_{1-6}$ alkyl, or 5- to 10-membered heteroaryl-$C_{1-6}$ alkyl, or

$R_1$ and $R_2$ and $R_3$ and $R_4$ are each independently linked to each other to form a 4- to 10-membered heterocycle containing the carbon and nitrogen atoms to which $R_1$ and $R_2$ and $R_3$ and $R_4$ are bonded.

Alternatively, said compound is a compound according to Chemical Formula 14, as defined hereinbelow.

Specifically, the compound represented by Chemical Formula 1-1 may be a synthetic small-molecule compound represented by the following Chemical Formulas 4 to 13, but is not limited thereto:

[Chemical Formula 4]

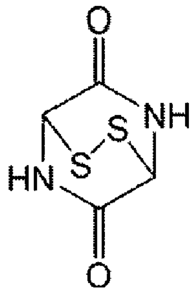

[Chemical Formula 5]

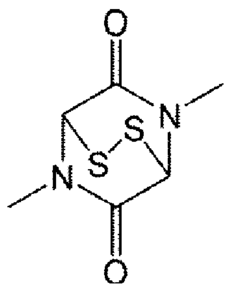

[Chemical Formula 6]

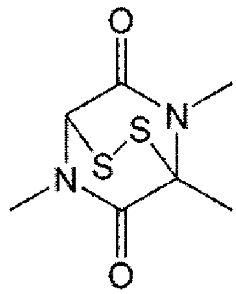

[Chemical Formula 7]

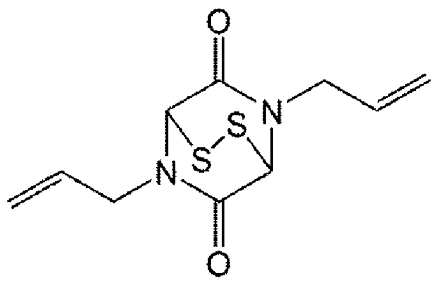

[Chemical Formula 8]

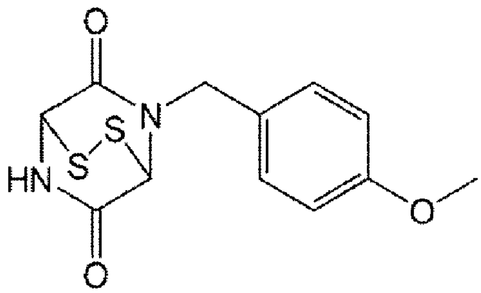

[Chemical Formula 9]

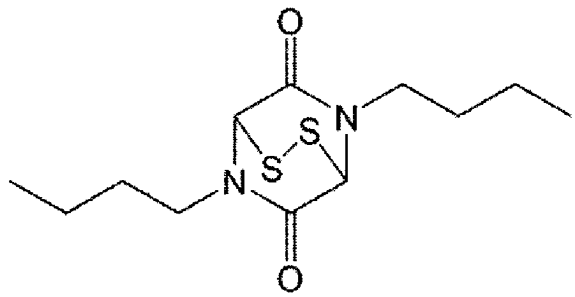

[Chemical Formula 10]

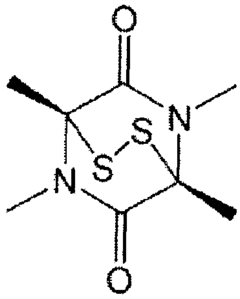

[Chemical Formula 11]

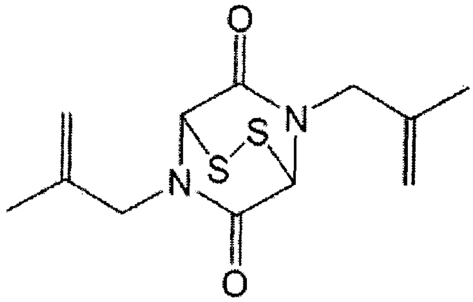

[Chemical Formula 12]

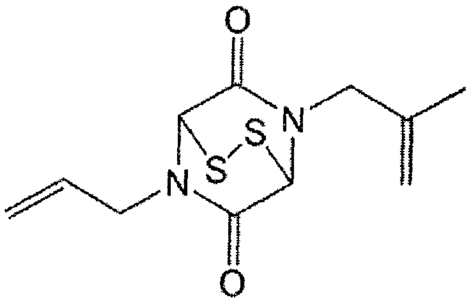

[Chemical Formula 13]

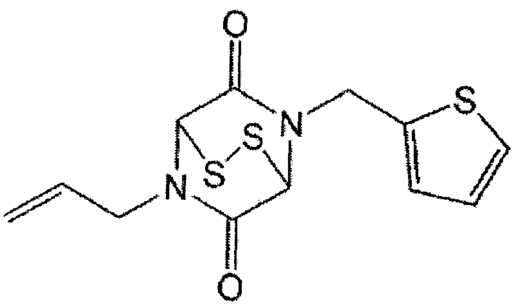

Alternatively, the active compound of the present invention may be a compound of

[Chemical Formula 14]

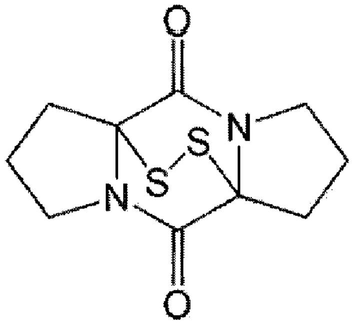

For example, the epidithiodioxopiperazine derivative compound comprised in the pharmaceutical composition of the present invention may be a compound represented by the following Chemical Formula 5, 7 or 14, while the compounds of Formulae 2 and 3 are reference compounds:

[Chemical Formula 2]

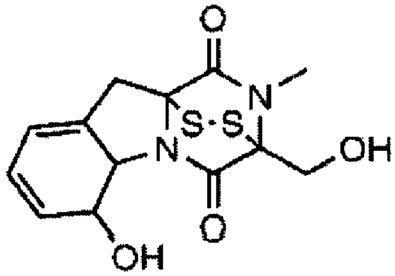

[Chemical Formula 3]

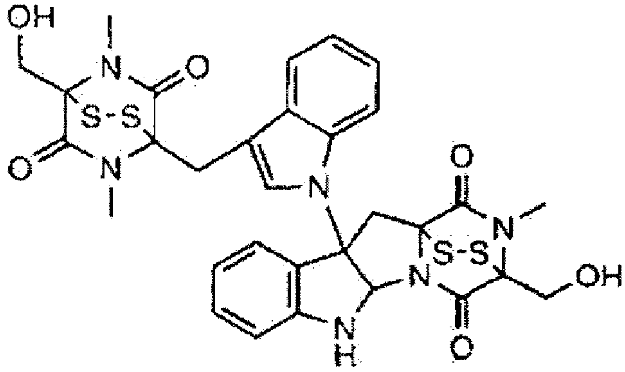

[Chemical Formula 5]

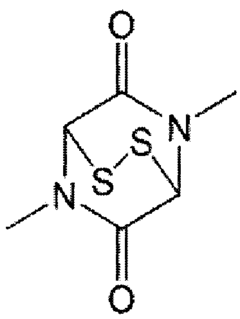

[Chemical Formula 7]

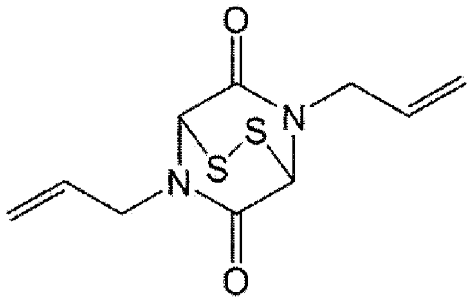

[Chemical Formula 14]

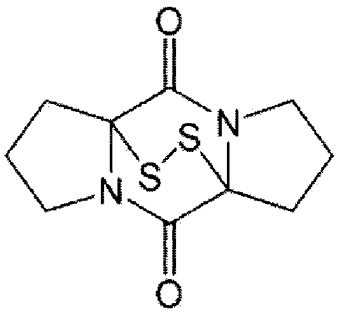

The derivative of the epidithiodioxopiperazine compound refers to a compound comprising an epidithiodioxopiperazine ring as a parent structure exhibiting activity. The derivative may be a compound in which the NH group or CH group in the compound ring represented by Chemical Formula 4 is substituted with various substituents known in the art, or may comprise a compound having a structure in which various compounds apparent to those skilled in the art are bonded to the NH group or CH group, but is not limited thereto. Modification and substitution of the structure of the compound represented by Chemical Formula 4 may be easily performed by those skilled in the art, for example, through the following process.

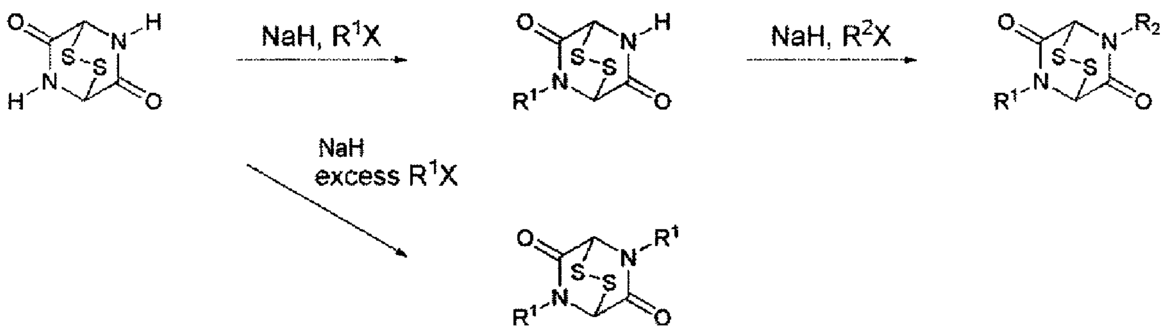


The compounds represented by Chemical Formulas 4 to 14 may be synthesized by those skilled in the art with reference to known methods, and used. For a specific synthesis method, reference is made to the method disclosed in Korean Patent Publication No. 10-1633975.

The composition of the present invention can achieve the effect of preventing or treating solid cancer by mimicking the intracellular activity of PrxII, but the specific mechanism of action is not limited thereto. For example, the epidithiodioxopiperazine derivative compound comprised in the composition of the present invention can mimic the activity of PrxII, and thus may exhibit anticancer activity by targeting cancer cells with low or no expression of peroxyredoxins, but is not limited thereto.

The solid cancer that can be prevented or treated using the composition of the present invention is selected from breast cancer, lung cancer, stomach cancer, colon cancer, prostate cancer, or pancreatic cancer.

As used herein, the term "pharmaceutically acceptable salt" refers to any salt that retains the desired biological and/or physiological activity of the compound or derivatives thereof and exhibits minimal undesired toxicological effects. In

the present invention, any salt can be used without limitation on the kind as long as the diketopiperazine ring comprising an intramolecular disulfide bridge is maintained. As the salt, an acid addition salt formed by a pharmaceutically acceptable free acid is useful. An acid addition salt is prepared by way of conventional methods, for example by dissolving the compound in an excessive amount of an acid aqueous solution and precipitating this salt using a water-miscible organic solvent such as methanol, ethanol, acetone, or acetonitrile. Equal molar amounts of compound and an acid or alcohol (for example, glycol monomethyl ether) in water may be heated, and then the mixture may be evaporated to dryness or the precipitated salt may be filtered under suction. In this case, inorganic acids and organic acids may be used as the free acid, and hydrochloric acid, hydrobromic acid, phosphoric acid, nitric acid, sulfuric acid, tartaric acid, and the like may be used as inorganic acids, and methane sulfonic acid, p-toluene sulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like may be used as organic acids, but the free acid is not limited thereto.

Pharmaceutically acceptable metal salts may be formed using bases. An alkali metal or alkaline earth metal salt is obtained, for example, by dissolving the compound in an excessive amount of an alkali metal hydroxide or alkaline earth metal hydroxide solution, filtering off the undissolved compound salt, and then evaporating the filtrate to dryness. In this case, as the metal salt, it is pharmaceutically suitable to prepare a sodium, potassium, or calcium salt, but the metal salt is not limited thereto. A silver salt corresponding to this salt may be obtained by reacting an alkali metal or alkaline earth metal salt with a suitable silver salt (for example, silver nitrate).

The pharmaceutically acceptable salts of the epidithiodioxopiperazine compound or derivatives thereof according to the present invention include all salts of acidic or basic groups that may exist unless otherwise indicated. For example, the pharmaceutically acceptable salts may include sodium, calcium, and potassium salts of hydroxyl groups, and the like, other pharmaceutically acceptable salts of amino groups include hydrobromides, sulfates, hydrogen sulfates, phosphates, hydrogen phosphates, dihydrogen phosphates, acetates, succinates, citrates, tartrates, lactates, mandelates, methanesulfonates (mesylates), and *p*-toluenesulfonates (tosylates), and these salts may be prepared by way of a method for preparing a salt known in the art.

The composition according to the present invention may further contain suitable carriers, excipients, and diluents commonly used in the preparation of pharmaceutical compositions. The composition is sterile or aseptic, and such solutions are sterile or aseptic, and the composition may contain water, buffers, isotonic agents, or other ingredients that are known to those skilled in the art and do not cause allergic or other adverse reactions when applied to animals or humans.

As used herein, the term "pharmaceutically acceptable carrier" includes any and all solvents, dispersion media, coatings, antibacterial agents, antifungal agents, isotonic agents, and the like. It is well known in the art to use the media and agents for pharmaceutically active substances. In addition to conventional media or agents that are immiscible with the active ingredient, the use thereof in therapeutic compositions is considered. Supplementary active ingredients may also be incorporated into the composition.

The composition may be prepared as a formulation such as a solution, emulsion, suspension, or cream, and may be used parenterally. The composition may be used in a conventional amount to prevent restenosis of blood vessels, and it is preferable that the amount of the composition used is differently applied depending on the patient's age, sex, condition, absorbed degree of the active ingredient in the body, inactivation rate, drugs used concurrently, and the like.

The present invention provides a pharmaceutical composition for use in a method for preventing or treating a solid cancer as defined in the claims, the method comprising administering the pharmaceutical composition to an individual in need thereof.

In the present invention, the term "prevention" refers to any action that inhibits or delays the onset of solid cancer by administration of the pharmaceutical composition according to the present invention, and the term "treatment" refers to any action that improves or advantageously changes symptoms caused by solid cancer by administration of the pharmaceutical composition.

In the present invention, the term "individual" refers to all animals, such as monkeys, cows, horses, sheep, pigs, chickens, turkeys, quails, cats, dogs, mice, rats, rabbits, or guinea pigs, including humans, which have or may develop solid cancer. By administering the pharmaceutical composition of the present invention to the individual, the disease may be effectively prevented or treated. The pharmaceutical composition of the present invention may be administered in parallel with known solid cancer therapeutic agents.

The pharmaceutical composition of the present invention is administered in a pharmaceutically effective amount. The term "pharmaceutically effective amount" refers to an amount sufficient to treat a disease at a reasonable benefit/risk ratio applicable to medical treatment and not to cause side effects, and the effective dosage level may be readily determined by those skilled in the art according to factors including patient's sex, age, weight, health status, disease severity, drug activity, sensitivity to drug, administration method, administration time, administration route and excretion rate, treatment period, and drugs used in combination or concomitantly and other factors well known in the medical arts.

As used herein, the term "administration" refers to providing a given substance to a patient by any suitable

method, and the composition of the present invention may be administered through any general route as long as it can reach the target tissue. The administration may be intraperitoneal administration, intravenous administration, intramuscular administration, subcutaneous administration, intradermal administration, oral administration, topical administration, intranasal administration, intrapulmonary administration, or rectal administration, but is not limited thereto. The pharmaceutical composition of the present invention may be administered by any device capable of transporting the active substance to the target cell. Preferred administration methods and preparations are intravenous injections, subcutaneous injections, intradermal injections, intramuscular injections, drip injections, and the like. Injections may be prepared using aqueous solvents such as physiological saline solution and Ringer's solution, non-aqueous solvents such as vegetable oil, higher fatty acid esters (for example, ethyl oleate), and alcohols (for example, ethanol, benzyl alcohol, propylene glycol, and glycerin), and the like, and may contain pharmaceutical carriers such as stabilizers to prevent degeneration (for example, ascorbic acid, sodium hydrogen sulfite, sodium pyrosulfite, BHA, tocopherol, and EDTA), emulsifiers, buffers for pH adjustment, and preservatives to inhibit the growth of microorganisms (for example, phenylmercuric nitrate, thimerosal, benzalkonium chloride, phenol, cresol, and benzyl alcohol).

For the prevention or treatment of solid cancer, the pharmaceutical composition of the present invention may further contain a known drug used for the prevention or treatment of known solid cancer, for example, a known anticancer agent other than the epidithiodioxopiperazine derivative compound or a pharmaceutically acceptable salt thereof as an active ingredient. The pharmaceutical composition of the present invention may be used concurrently with other treatments known for the treatment of these diseases. Other treatments include, but are not limited to, chemotherapy, radiation therapy, hormone therapy, bone marrow transplantation, stem-cell replacement therapy, other biological therapies, immunotherapy, and the like.

**[Mode for Invention]**

Hereinafter, the present invention will be described in more detail with reference to Examples. These Examples are only for illustrating the present invention more specifically, and the scope of the present invention is defined by the claims.

**Experimental Example 1: Cell line and culture conditions**

From American type culture collection (ATCC), breast cancer cell lines MDA-MB-231 and Hs-578T; lung cancer cell lines A549 and H460; stomach cancer cell lines MKN28 and MKN74; skin cancer cell lines SK-MEL-5 and SK-MEL-28; colon cancer cell lines RKO and HCT116; a prostate cancer cell line PC3; and a pancreatic cancer cell line Panc-1; a normal breast cell line MCF-10A; a normal lung fibrous cell line IMR90; and a normal colon cell line CCD-841 were procured. The Hs-578T, H460, MKN28, and MKN74 cells were cultured in high-glucose RPMI 1640 supplemented with 10% fetal bovine serum (FBS, Hyclone), 100 U/mL penicillin, and 100 U/mL streptomycin, and the MDA-MB-231, A549, PC3, and Panc-1 cells were cultured in high-glucose DMEM (Dulbecco's Modified Eagle Medium) supplemented with 10% FBS, 100 U/mL penicillin, and 100 U/mL streptomycin. The SK-MEL-5 and SK-MEL-28 cells were cultured in high-glucose EMEM (Eagle's Minimum Essential Medium) supplemented with 10% FBS, 100 U/mL penicillin, and 100 U/mL streptomycin, and the IMR90 and CCD-841 cells were cultured in high-glucose MEM (Minimum Essential Medium) supplemented with 10% FBS, 100 U/mL penicillin, and 100 U/mL streptomycin. The RKO cells were cultured in high-glucose McCoy supplemented with 10% FBS, 100 U/mL penicillin, and 100 U/mL streptomycin, and the MCF-10A cells were cultured in DMEM F12 supplemented with 10% FBS, 100 U/mL penicillin, and 100 U/mL streptomycin. These cells were sub-cultured at 37°C in a 5% $CO_2$ atmosphere.

**Experimental Example 2: Cell proliferation assay**

The cells ($2\times10^3$ cells/well) were cultured in 96-well plates for 24 hours, and then treated with the compound of the present invention for 2 hours. The cells were placed in fresh complete media and cultured for the labelled time. For cell proliferation, the cells were treated with WST-1 reagent for 1 hour, and the absorbance of the formazan product at 450 nm was measured using a UV/VIS ELISA reader.

**Experimental Example 3: Cell viability assay**

In order to determine the $IC_{50}$ value for examining cytotoxicity, the cells ($2\times10^5$ cells/well) were aliquoted into 96-well plates and treated with the labelled compounds at various concentrations for 24 hours. The cell viability was measured using the Cell Titer-Glo Luminescence Assay Reagent Kit (Promega, G7570), and the luminescence was read using an Envision plate reader. The percentage of cell growth with respect to that in the vehicle control group (0.1% DMSO-treated cells) was calculated.

**Experimental Example 4: Cell cycle assay**

The cells ($2\times10^5$ cells/well) were cultured in 6-well plates for 24 hours, and then treated with the compounds of the present invention for 2 hours. The cells were placed in fresh complete media and further cultured for 24 hours. The cells were fixed overnight in 70% ethanol at 4°C, washed with cold PBS, and resuspended in PBS solution containing RNase A (10 μg/mL) at 37°C for 30 minutes. The suspended cells were stained with propidium iodide (PI, 50 μg/mL, Invitrogen) for 5 minutes, and flow cytometry (FACS Calibur, BD) was performed. The cell population was analyzed using ModiFit LT software.

**Experimental Example 5: Migration assay**

The cells ($2\times10^5$ cells/well) were cultured in 6-well plates for 24 hours, and then treated with the compounds of the present invention for 2 hours. After treatment, the cells were serum-starved by further culturing in fresh culture medium containing 0.5% FBS for 12 hours. The serum-starved cells ($5\times10^4$ cells/well) were transferred to the upper chamber (8 μm pore size) of a 24-well trans-well culture plate (Costar) with the bottom layer pre-coated with 0.1% gelatin B (Sigma Aldrich). The lower chamber was filled with culture medium containing 20% FBS. Twenty-four hours after plating, non-migrated cells in the upper chamber were removed. After the cells which had not migrated from the upper chamber were removed, the upper chamber was fixed with methanol and stained with 0.6% hematoxylin (DAKO). The stained cells were photographed and counted. The number of migratory cells was determined by averaging the values from three wells.

**Experimental Example 6: Colony formation assay**

The cells ($2\times10^3$ cells/well) were cultured in 6-well plates for 24 hours, and then treated with the compounds of the present invention for 2 hours. The cells were placed in fresh complete media and additionally cultured for 10 days. The cells were washed with PBS and stained with crystal violet. Blue-stained colonies were manually counted.

**Experimental Example 7: Mouse xenograft assay**

The A549, SK-MEL-28, MKN-28, and Luc-MDA-MB-231 cells ($2\times10^6$ cells) were suspended in 100 μL of Matrigel solution (BD Biosciences) and injected subcutaneously into 5-week-old male mice. After 7 days, mice were intraperitoneally injected with 100 μL of vehicle control group (0.1% DMSO) and the labelled compound (300 μg/kg) every 3 days for 20 days. For A549, SK-MEL28, and MKN28-derived tumors, the caliper measurement of width (W) and length (L) of xenografted tumors was performed and the tumor volume was calculated by the following equation: Volume = $[L\times W^2]/2$. For tumors induced with the Luc-MDA-MB-231 cells, the tumor volume was measured by 2D optical topography. Briefly, mice were intraperitoneally injected with D-luciferin solution (150 mg/kg, Caliper Life Sciences), and anesthetized with 2% isoflurane 10 minutes later, and then images were taken for 10 seconds using the IVIS 200 system (Xenogen).

**Experimental Example 8: Immunohistochemistry and immunofluorescence**

A tumor tissue specimen embedded in a paraffin block was cut to a thickness of 4 μm. The tissue sections were deparaffinized according to standard protocols and rehydrated with a series of graded alcohols. The antigen was immersed in an antigen unmasking solution (VECTOR, H-3300-250) at 95°C for 15 minutes, and washed with PBS when the temperature dropped to room temperature. Endogenous peroxidase activity was quenched with BLOXALL Endogenous Blocking Solution, and the tissue sections were incubated for 30 minutes. After a blocking step of 1 hour with a blocking solution containing normal goat serum in 0.1% Triton X-100, the tissue sections were reacted with anti-Ki67 antibody (1:100, Invitrogen, PA1-38032) at room temperature for 2 hours. For immunohistochemistry (IHC), slides were washed with PBS three times, and incubated with biotinylated secondary antibody (1:200) in 0.1% Triton X-100 at room temperature for 1 hour. ImmPACT DAB substrate (VECTOR, SK-4105) was added to the slides to develop positive color. For immunofluorescence (IF), the washed slides were incubated with fluorophore-conjugated secondary antibody (1:200) in 0.1% Triton X-100 for 1 hour. Fluorescence was visualized and imaged using a confocal microscope (Nikon A1R).

**Experimental Example 9: TUNEL Assay**

The Luc-MDA-MB-231 and MKN-28 xenograft tumor samples were surgically separated, then put into an OCT (optimal cutting temperature) compound solution, and frozen and embedded on dry ice for 10 minutes, and then the frozen samples were cut to a thickness of 10 μm. The tissue sections were washed with distilled water for 10 minutes and fixed with 4% formalin for 20 minutes. Thereafter, the tissue sections were washed with PBS for 30 minutes, and incubated in

permeabilization solution (0.1% Triton X-100) for 5 minutes. Apoptotic cells were visualized using the *In situ* Cell Death Detection Kit (Roche, 11684795910) according to the manufacturer's instructions. The cell images were acquired through a microscope (Zeiss LSM880 Airyscan).

**Experimental Example 10: Patient-derived tumor xenograft experiment**

Animal experiments were approved by the Institutional Animal Care and Use Committee (IACUC) of Seoul National University College of Medicine and complied with ARRIVE (Animal Research: Reporting of *In Vivo* Experiments) guidelines. Patient-derived lung tumors were subcutaneously implanted into the backs of 4-week-old and 17-week-old NSG (NOD-scid-IL2Rgnull) and humanized NSG (HuNSG) mice, respectively. The compound represented by Chemical Formula 7 (A5, 300 mg/kg) and/or pembrolizumab (humanized anti-PD-1 antibody, 5 mg/kg) dissolved in PBS were intraperitoneally injected to the mice every 3 days for 3 weeks and every 5 days for 3 weeks, respectively. The tumors were allowed to grow for 28 days, excised, and quantified. The tumor volume was measured every 3 days using Vernier calipers and calculated using the following equation.

$$V = a \times b^2/2$$

In the equation, *a* and *b* mean the superficial diameter of major axis and the superficial diameter of minor axis, respectively.

**Example 1** (reference): **Cytotoxicity and cancer cell proliferation inhibitory effect of natural epidithiodioxopiperazine derivative**

Among the cancer cells prepared according to Experimental Example 1, breast cancer cell lines MDA-MB-231 and Hs-578T were treated with DMSO as a control vehicle and a natural epidithiodioxopiperazine derivative gliotoxin, the cytotoxicity and proliferation rate at each concentration of the treated compound were calculated according to Experimental Example 2, and the results are illustrated in Figure 1.

As illustrated in Figure 1(A), a high cell viability of 80% or more is acquired up to a concentration of about 200 nM when the cells are treated with gliotoxin and chetomin. This suggests that these compounds are not cytotoxic at the corresponding concentrations, and thus concentrations within the above range were selectively used in the following experiments to confirm the pharmacological activity.

Meanwhile, as illustrated in Figures 1(B) and 1(C), the proliferation rate in cells treated with gliotoxin (100 nM) or chetomin (50 nM) was significantly decreased as compared to that in DMSO-treated cells, the control group. This indicates that the natural epidithiodioxopiperazine derivative effectively inhibits cancer cell proliferation.

**Example 2** (reference): **Cell cycle progression inhibitory effect of natural epidithiodioxopiperazine derivative on cancer cell**

Among the cancer cells prepared according to Experimental Example 1, breast cancer cell lines MDA-MB-231 and Hs-578T were not treated or were treated with a natural epidithiodioxopiperazine derivative gliotoxin, the cell cycle was analyzed and the cell percentage in G1 phase was calculated according to Experimental Example 4, and the results are illustrated in Figure 2.

As illustrated in Figure 2, when the cancer cell lines to which gliotoxin is added are cultured, it has been confirmed that the cell percentage in G1 phase is increased by 10% or more as compared to that in the untreated group.

**Example 3** (reference): **Migratory activity inhibitory effect of natural epidithiodioxopiperazine derivative on cancer cell**

Among the cancer cells prepared according to Experimental Example 1, breast cancer cell lines MDA-MB-231 and Hs-578T were treated with DMSO as a control vehicle and natural epidithiodioxopiperazine derivative gliotoxin, cell migration to the FBS-containing medium was observed and the number of migrated cells was counted according to Experimental Example 5, and the results are illustrated in Figures 3 and 4.

As illustrated in Figures 3 and 4, the migration of breast cancer cells induced to the serum-containing medium is significantly decreased when the cancer cell lines are treated with gliotoxin and chetomin.

**Example 4: Selective cancer cell killing effect of synthetic epidithiodioxopiperazine derivative**

The cytotoxic effect of the synthetic epidithiodioxopiperazine derivative of the present invention on various cancer cells and normal cells was examined, and the results are illustrated in Figure 5. As illustrated in Figure 5, the synthetic epidithiodioxopiperazine derivative of the present invention has significantly lower $IC_{50}$ values in various cancer cell lines than in three normal cells (MCF-10A, IMR90, and CCD-841), and this indicates that the synthetic epidithiodioxopiperazine derivative selectively kills cancer cells rather than normal cells.

**Example 5: Colony formation inhibitory effect of synthetic epidithiodioxopiperazine derivative on cancer cell**

Among the cancer cells prepared according to Experimental Example 1, breast cancer cell lines MDA-MB-231 and Hs-578T; lung cancer cell lines A549 and H460; stomach cancer cell lines MKN28 and MKN74; skin cancer cell lines SK-MEL-5 and SK-MEL-28; colon cancer cell lines RKO and HCT116; a prostate cancer cell line PC3; and a pancreatic cancer cell line Panc-1 were treated with DMSO as a control vehicle and synthetic epidithiodioxopiperazine derivatives A2, A5, and A10. The killing activity of the epidithiodioxopiperazine derivatives on these cancer cells were observed with the naked eye and the number of colonies formed was counted according to Experimental Example 6, and the results are illustrated in Figures 6 to 12.

As illustrated in Figures 6 to 12, although there is a difference in degree, it has been found that all of A2, A5, and A10 efficiently inhibit colony formation by these cancer cells at low concentrations (50 nM and 200 nM) at which A2, A5, and A10 do not exhibit non-specific cytotoxicity. For example, colony formation was inhibited in most cancer cells even at a concentration of 50 nM in the case of A10 compound. However, in the case of A2 and A5, the colony formation inhibitory effect is equal to or somewhat superior to that in the control vehicle-treated group when the cancer cells are treated with A2 and A5 at a concentration of 50 nM, but the colony formation inhibitory effect is remarkably so as to be observed with the naked eye in all tested cells when the concentrations of A2 and A5 are increased to 200 nM.

**Example 6: Growth inhibitory effect of synthetic epidithiodioxopiperazine derivative on xenograft**

In the mouse xenograft models prepared by injecting various cancer cell lines according to Experimental Example 7, the tumor growth inhibitory effect by the treatment with the synthetic epidithiodioxopiperazine derivative of the present invention was examined, and the results are illustrated in Figure 13. Specifically, the tumor size was measured while the compound of the present invention was administered for 30 days, and on the final 30th day, the mice were killed, the tumors were excised, and the volume and weight thereof were measured. As a control group, mice administered with DMSO as a vehicle instead of the compound of the present invention were used. As illustrated in Figure 13, the growth of all xenograft tumors by lung cancer, skin cancer, stomach cancer and breast cancer cell lines was greatly inhibited by treatment with the compound of the present invention, and the size of the formed tumor was significantly smaller than that in the control group. This indicates that the compound of the present invention can effectively inhibit the growth of tumors.

**Example 7: *In vivo* cancer cell proliferation inhibitory effect of synthetic epidithiodioxopiperazine derivative**

Using the immunohistochemistry, immunofluorescence and TUNEL assays according to Experimental Examples 8 and 9, the *in vivo* cancer cell proliferation inhibitory effect of the synthetic epidithiodioxopiperazine derivative was examined, and the results are illustrated in Figure 15. As illustrated in Figure 15, in the case of being treated with the compound of the present invention, the expression of Ki-67, which is involved in proliferating ability of tumor, is significantly decreased in both breast cancer and stomach cancer cell lines as compared to that in the control group. In contrast, in TUNEL staining to measure apoptosis of cancer cells, apoptosis is not observed in both the vehicle control group and the case of being treated with the compound of the present invention. The accuracy of the TUNEL assay has been verified by artificially inducing DNA damage by treating the tissue sections with DNase (Figure 15G). This suggests that the compound of the present invention may exhibit a preventive or therapeutic effect on cancer diseases by effectively inhibiting proliferation rather than apoptosis of cancer cells.

**Example 8: Tumor growth inhibitory effect of synthetic epidithiodioxopiperazine derivative**

While the mouse models prepared by implanting a patient-derived lung adenocarcinoma tumor according to Experimental Example 10 were not treated, were treated only with a representative synthetic epidithiodioxopiperazine derivative A5, or were concurrently treated with A5 and pembrolizumab, a humanized antibody known to be used for cancer immunotherapy, the changes in volume of the tumor were periodically observed, and the results are illustrated in Figure 15.

As illustrated in Figure 15, the tumor volume increases as time elapses in the case of the non-treated control

group in NSG mice, but the increase in tumor volume is suppressed in the A5-treated group. In the case of administrating A5 to humanized NSG mice (HuNSG), an effect of decreasing the tumor size is exerted as compared to the untreated control group, as well as the group treated only with A5 is superior to the pembrolizumab-treated group or the experimental group concurrently treated with A5 and pembrolizumab in the effect of inhibiting tumor growth and decreasing the tumor size.

**Claims**

1. A pharmaceutical composition for use in prevention or treatment of solid cancer comprising as an active ingredient a compound represented by the following Chemical Formula 1-1 comprising an intramolecular disulfide bridge in an epidithiodioxopiperazine ring or a pharmaceutically acceptable salt thereof, or represented by Chemical Formula 14, or a pharmaceutically acceptable salt thereof

   wherein the solid cancer is breast cancer, lung cancer, stomach cancer, prostate cancer, or pancreatic cancer:

   [Chemical Formula 1-1]

   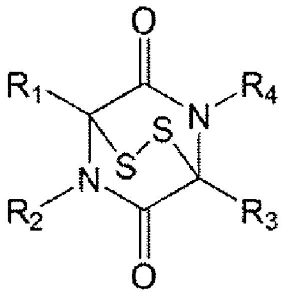

   wherein in Chemical Formula 1-1;
   $R_1$ to $R_4$ are each independently hydrogen, linear or branched $C_{1-6}$ alkyl, alkenyl, alkynyl, $C_{1-6}$ alkoxy-aryl-$C_{1-6}$ alkyl, or 5- to 10-membered heteroaryl-$C_{1-6}$ alkyl, and wherein
   the compound represented by Chemical Formula 14 has the following structure:

   [Chemical Formula 14]

   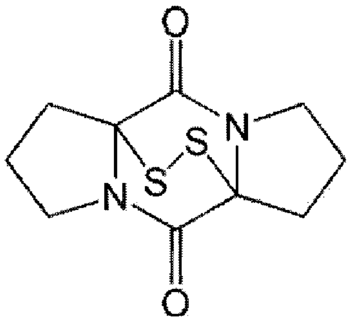

2. The composition for use according to claim 1, wherein the compound represented by Chemical Formula 1-1 or Chemical Formula 14 is any one or more selected from compounds represented by the following Chemical Formulas 4 to 14:

   [Chemical Formula 4]

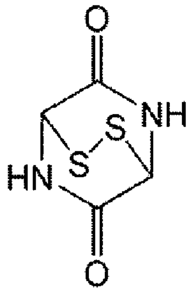

[Chemical Formula 5]

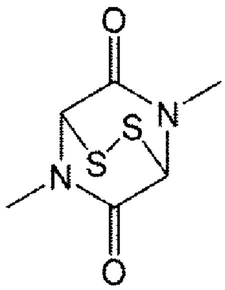

[Chemical Formula 6]

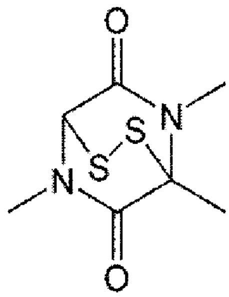

[Chemical Formula 7]

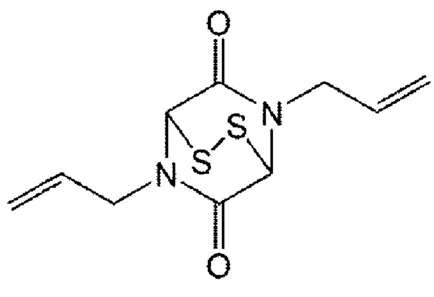

[Chemical Formula 8]

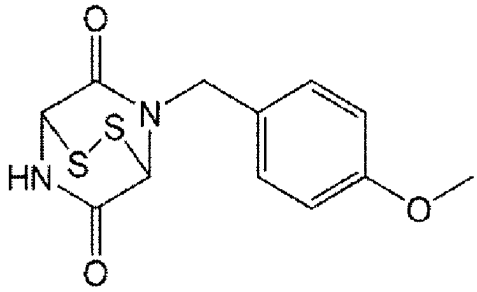

[Chemical Formula 9]

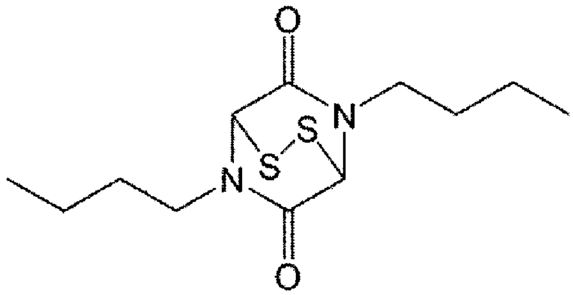

[Chemical Formula 10]

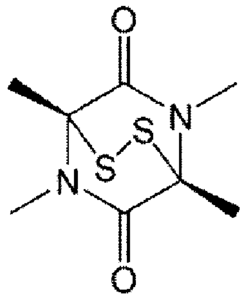

[Chemical Formula 11]

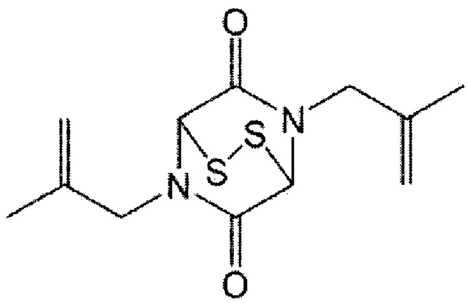

[Chemical Formula 12]

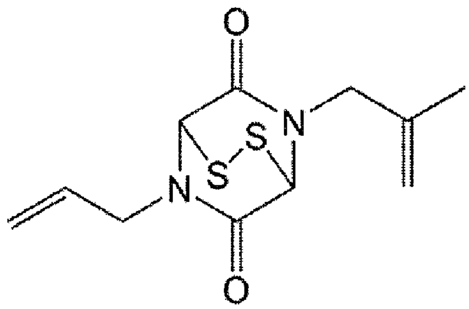

[Chemical Formula 13]

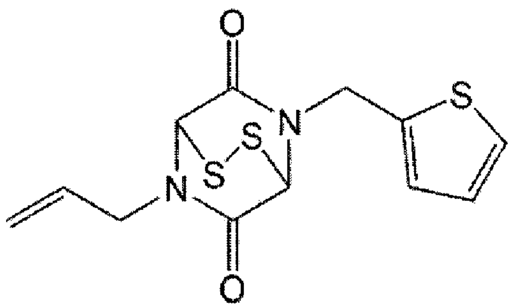

[Chemical Formula 14]

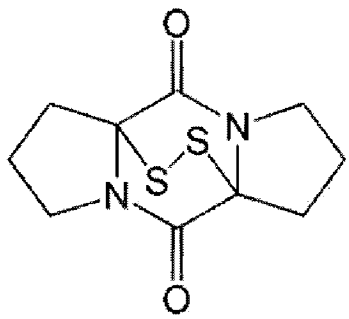

3. The composition for use according to claim 1, wherein the compound is a compound represented by the following Chemical Formula 5, 7, or 14:

[Chemical Formula 5]

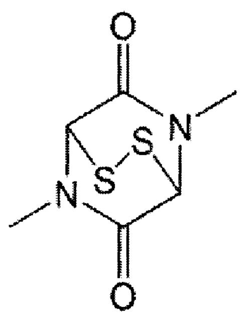

[Chemical Formula 7]

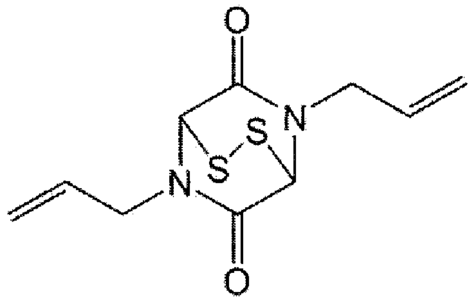

[Chemical Formula 14]

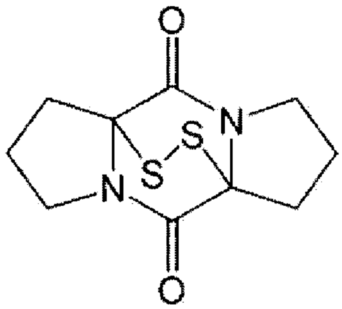

.

## Patentansprüche

1. Pharmazeutische Zusammensetzung zur Vorbeugung oder Behandlung von solidem Krebs, umfassend eine Verbindung, dargestellt durch die folgende chemische Formel 1-1, umfassend eine intramolekulare Disulfidbrücke in einem Epidithiodioxopiperazinring, oder ein pharmazeutisch unbedenkliches Salz davon, oder dargestellt durch die chemische Formel 14 oder ein pharmazeutisch unbedenkliches Salz davon, als Wirkstoff

   wobei der solide Krebs Brustkrebs, Lungenkrebs, Magenkrebs, Prostatakrebs oder Bauchspeicheldrüsenkrebs ist:

   [Chemische Formel 1-1]

   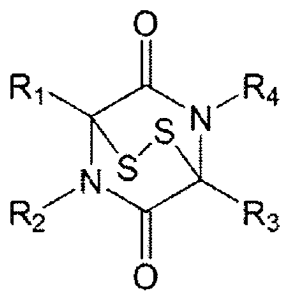

   wobei in der chemischen Formel 1-1:
   $R_1$ bis $R_4$ jeweils unabhängig voneinander Wasserstoff, lineares oder verzweigtes $C_{1-6}$-Alkyl, -Alkenyl, -Alkinyl, $C_{1-6}$-Alkoxyaryl-$C_{1-6}$-alkyl- oder 5- bis 10-gliedriges Heteroaryl-$C_{1-6}$-Alkyl sind, und wobei die durch die chemische Formel 14 dargestellte Verbindung die folgende Struktur aufweist:

[Chemische Formel 14]

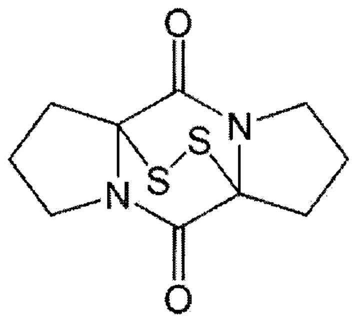

.

**2.** Zusammensetzung zur Verwendung nach Anspruch 1, wobei die durch die chemische Formel 1-1 oder die chemische Formel 14 dargestellte Verbindung eine oder mehrere ist, ausgewählt aus durch die folgenden chemischen Formeln 4 bis 14 dargestellten Verbindungen:

[Chemische Formel 4]

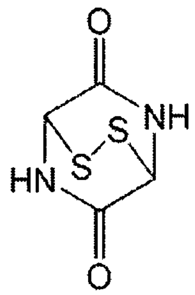

[Chemische Formel 5]

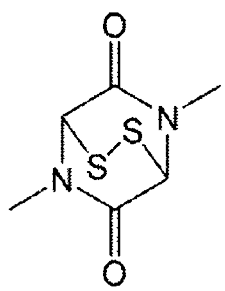

[Chemische Formel 6]

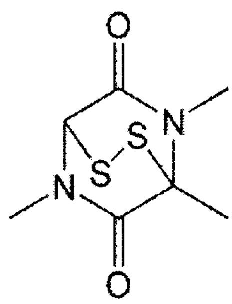

[Chemische Formel 7]

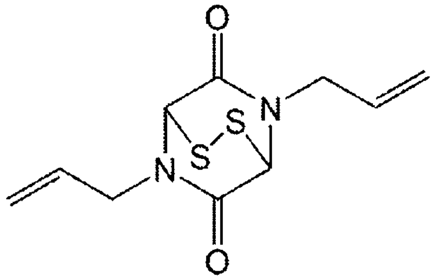

[Chemische Formel 8]

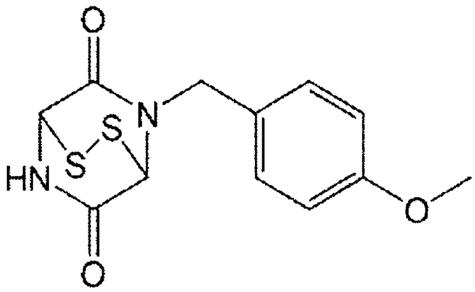

[Chemische Formel 9]

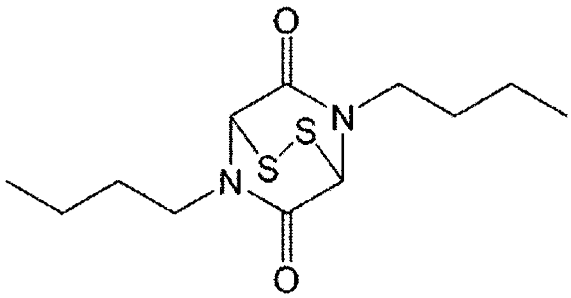

[Chemische Formel 10]

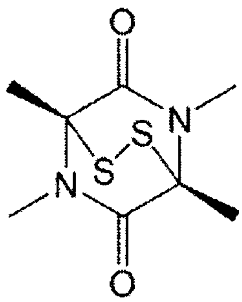

[Chemische Formel 11]

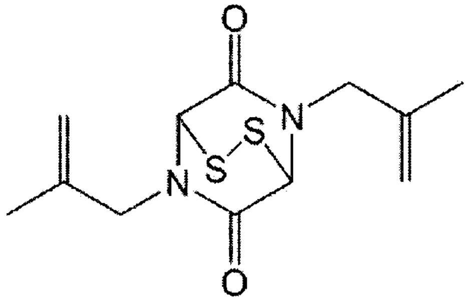

[Chemische Formel 12]

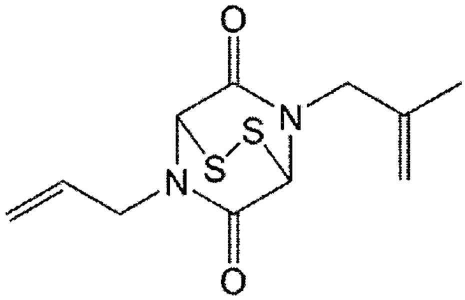

[Chemische Formel 13]

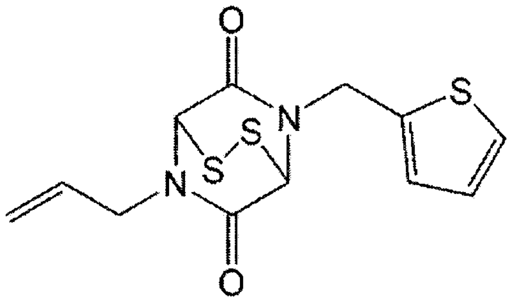

[Chemische Formel 14]

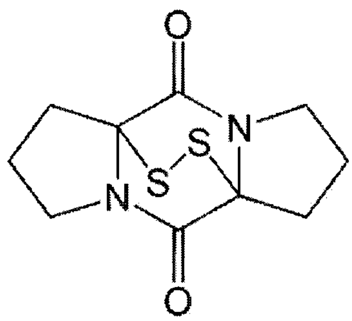

.

**3.** Zusammensetzung zur Verwendung nach Anspruch 1, wobei die Verbindung eine Verbindung ist, dargestellt durch die folgende chemische Formel 5, 7 oder 14:

[Chemische Formel 5]

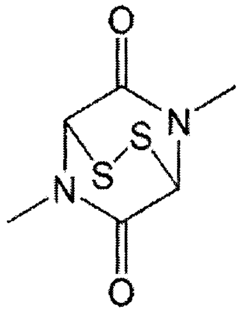

[Chemische Formel 7]

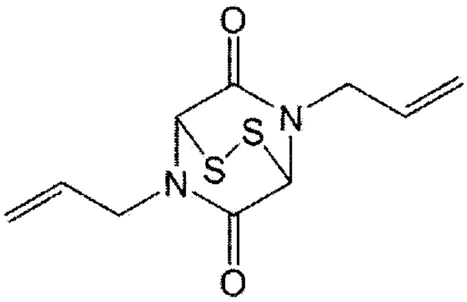

[Chemische Formel 14]

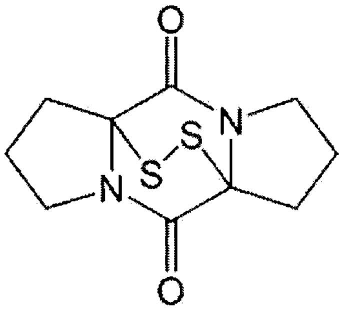

**Revendications**

1. Unecomposition pharmaceutique destinée à la prévention ou au traitement des tumeurs solides, comprenant comme principe actif un composé représenté par la Formule Chimique 1-1, comprenant un pont disulfure intramoléculaire dans un cycle épidithiodioxopipérazine, ou un sel pharmaceutiquement acceptable de celui-ci, ou représenté par la Formule Chimique 14, ou un sel pharmaceutiquement acceptable de celui-ci

   dans lequel le cancer solide est un cancer du sein, un cancer du poumon, un cancer de l'estomac, un cancer de la prostate ou un cancer du pancréas :

[Formule Chimique 1-1]

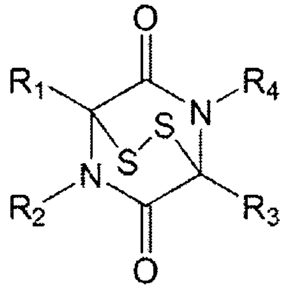

dans lequel dans la Formule Chimique 1-1 ;

$R_1$ à $R_4$ représentent chacun indépendamment un atome d'hydrogène, un groupe alkyle en $C_{1-6}$ linéaire ou ramifié, un alcényle, un alcynyle, un alcoxy-aryl-alkyleen $C_{1-6}$ $C_{1-6}$ ou un hétéroaryl-alkyle en $C_{1-6}$ à 5 à 10 chaînons, et dans lequel

le composé représenté par la Formule Chimique 14 possède la structure suivante :

[Formule Chimique 14]

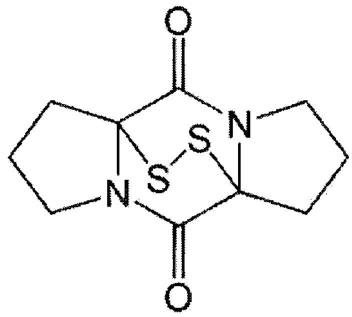

.

2. Composition à utiliser selon la revendication 1, dans laquelle le composé représenté par la formule chimique 1-1 ou la Formule Chimique 14 est un ou plusieurs composés choisis parmi les composés représentés par les Formules Chimiques 4 à 14 suivantes :

[Formule Chimique 4]

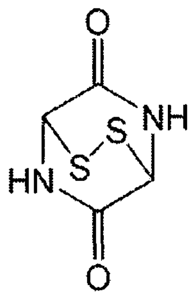

[Formule Chimique 5]

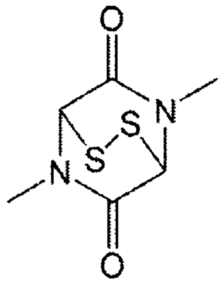

[Formule Chimique 6]

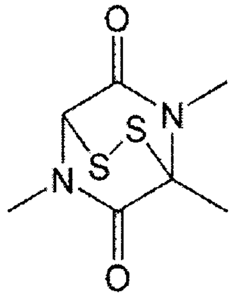

[Formule Chimique 7]

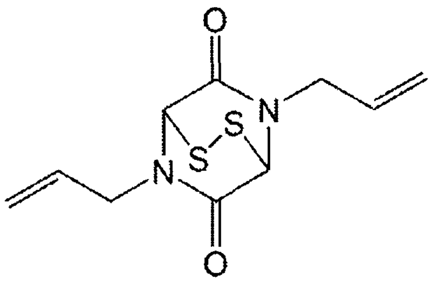

[Formule Chimique 8]

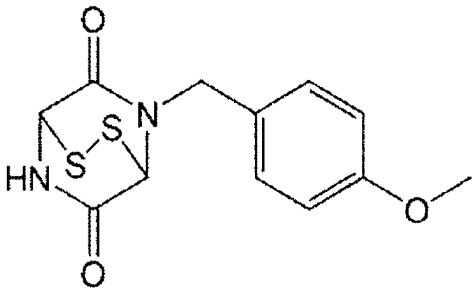

[Formule Chimique 9]

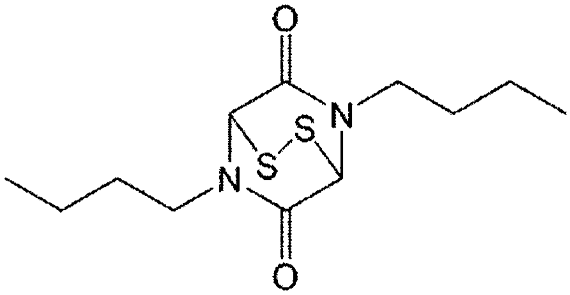

[Formule Chimique 10]

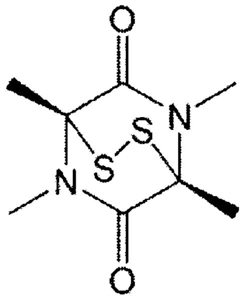

[Formule Chimique 11]

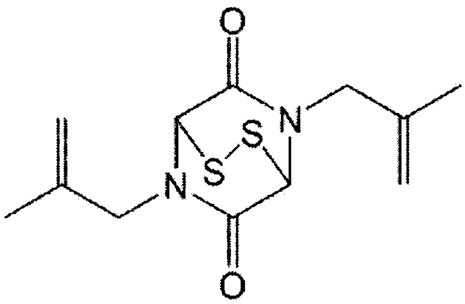

[Formule Chimique 12]

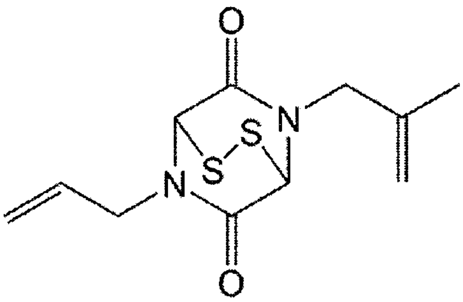

[Formule Chimique 13]

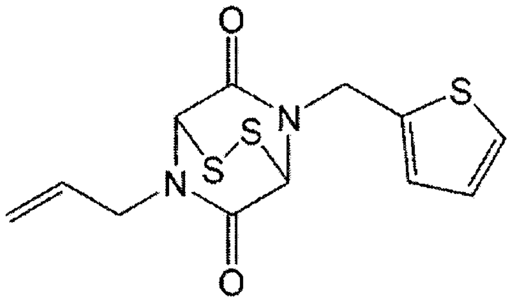

[Formule Chimique 14]

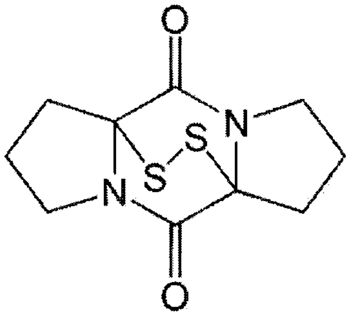

3. Composition à utiliser selon la revendication 1, dans laquelle le composé est un composé représenté par la Formule Chimique suivante 5, 7 ou 14 :

[Formule Chimique 5]

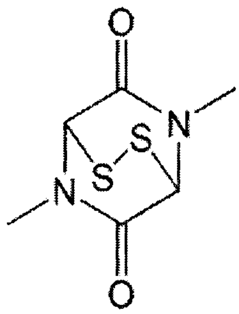

[Formule Chimique 7]

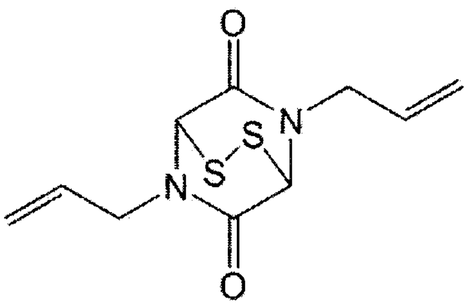

[Formule Chimique 14]

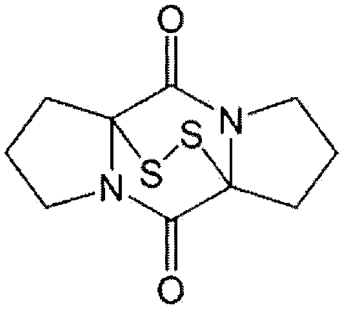

.

[Figure 1]

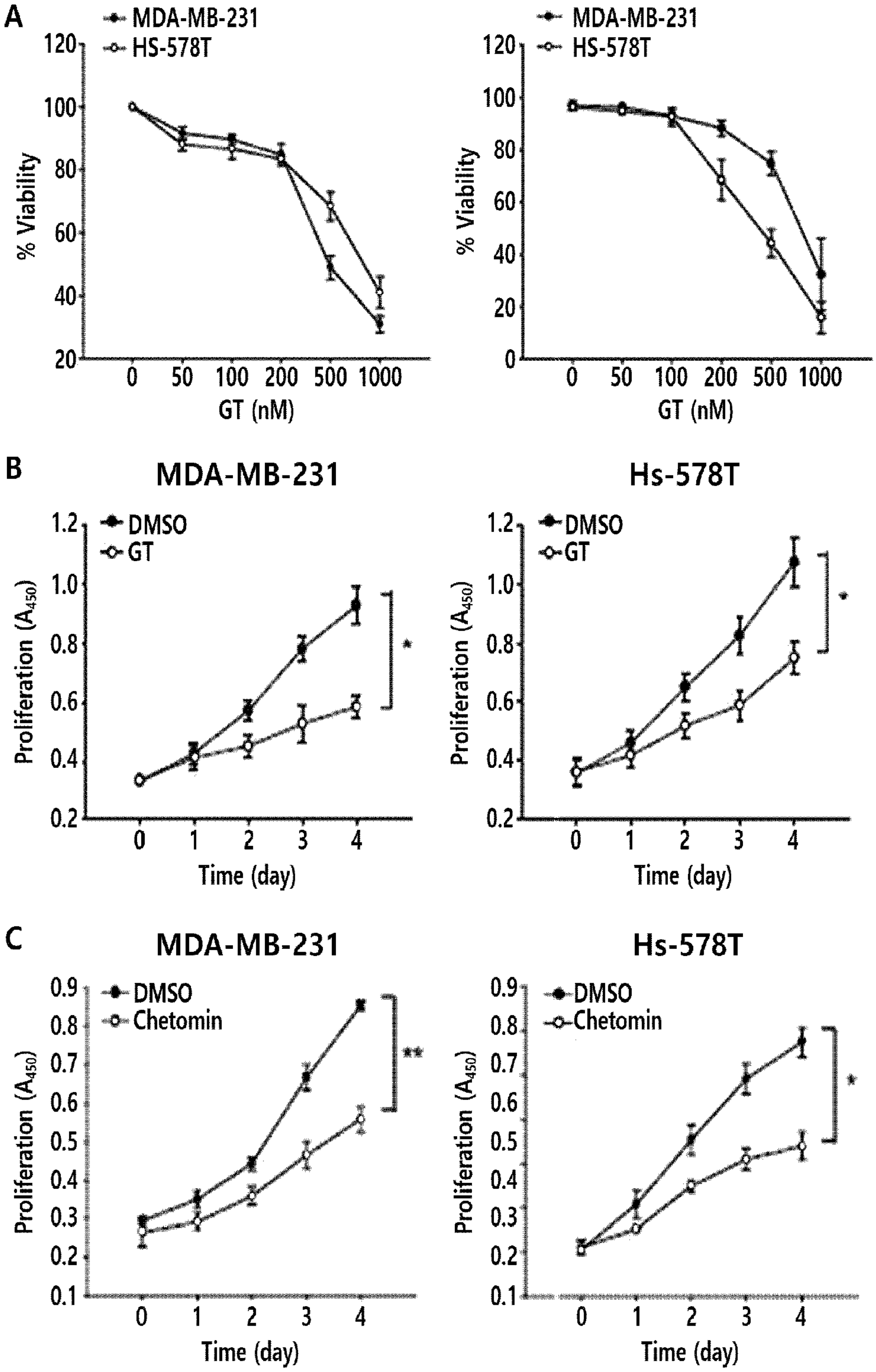
A
MDA-MB-231
HS-578T
% Viability
GT (nM)
0 50 100 200 500 1000
B
MDA-MB-231
Hs-578T
DMSO
GT
Proliferation ($A_{450}$)
Time (day)
*
C
MDA-MB-231
Hs-578T
DMSO
Chetomin
Proliferation ($A_{450}$)
Time (day)
**
*

[Figure 2]

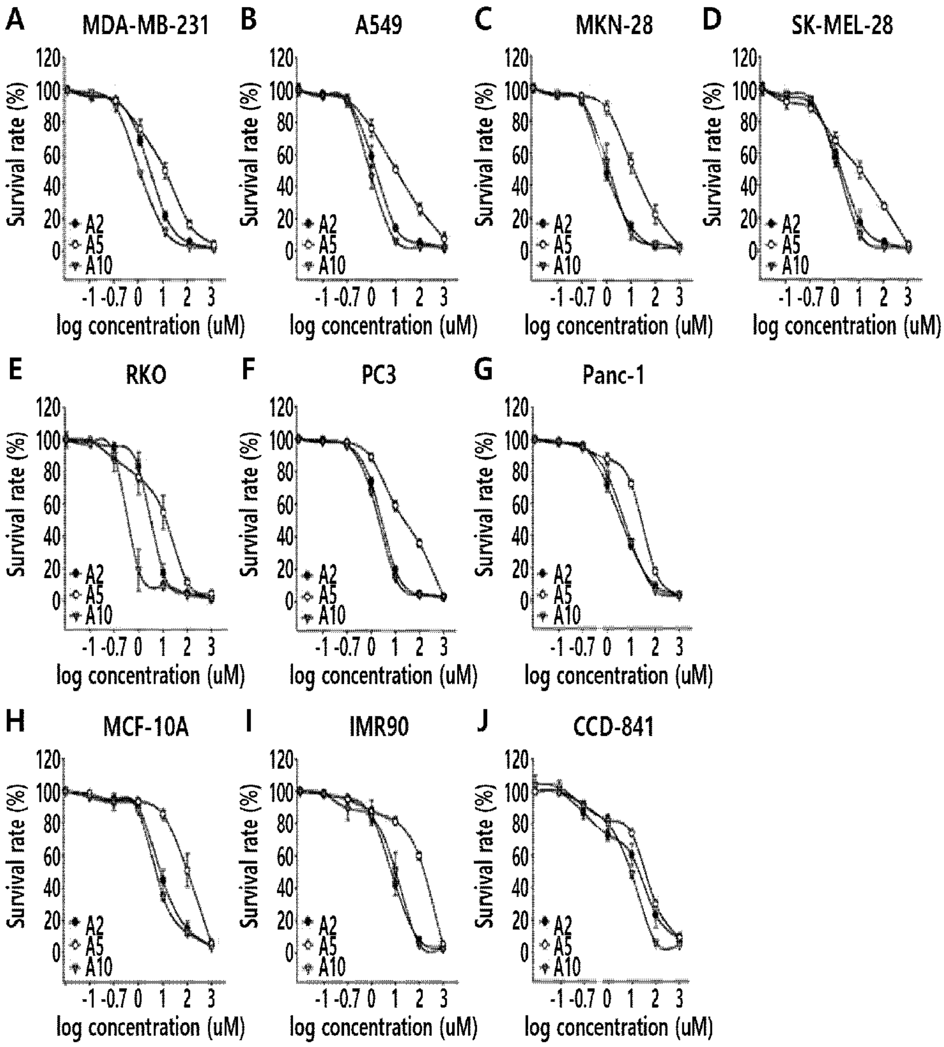


K

| Drug / Cell line | A2 | A5 | A10 |
|---|---|---|---|
| | IC$_{50}$(μM) | | |
| MDA-MB-231 | 2.214 | 8.252 | 0.7468 |
| A549 | 1.205 | 8.17 | 0.7016 |
| MKN28 | 0.7844 | 13.7 | 1.01 |
| SK-MEL-28 | 1.422 | 10.58 | 1.085 |
| RKO | 2.909 | 12.69 | 0.2498 |
| PC3 | 2.238 | 16.81 | 1.66 |
| Panc-1 | 3.255 | 27.26 | 4.852 |
| MCF-10A | 7.615 | 122 | 5.255 |
| IMR90 | 6.907 | 211.1 | 11.04 |
| CCD-841 | 16.82 | 77.78 | 10.74 |

[Figure 3]

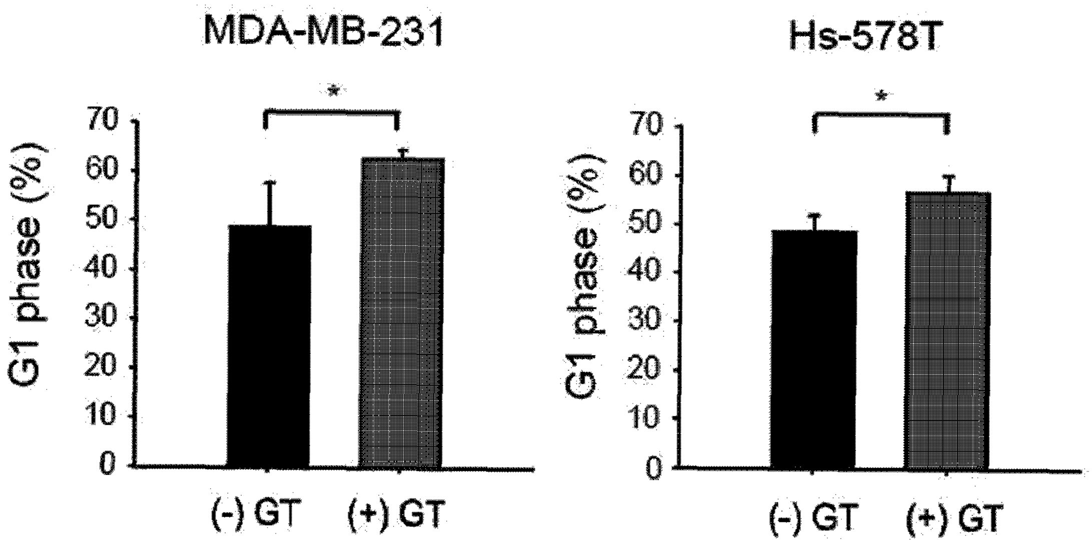

[Figure 4]

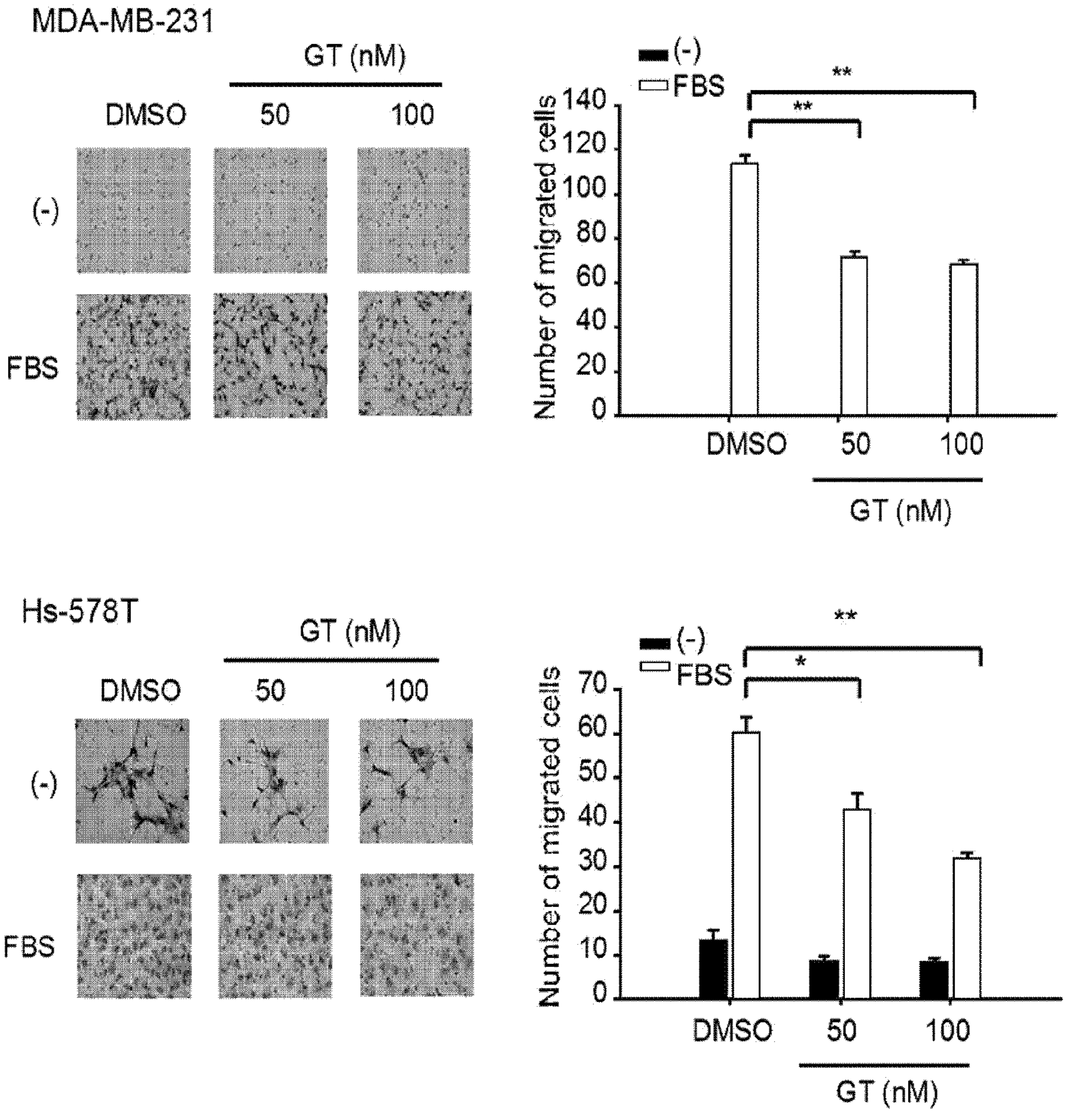
MDA-MB-231
GT (nM)
DMSO
50
100
(-)
FBS
(-)
FBS
**
**
Number of migrated cells
140
120
100
80
60
40
20
0
DMSO
50
100
GT (nM)
Hs-578T
GT (nM)
DMSO
50
100
(-)
FBS
(-)
FBS
**
*
Number of migrated cells
70
60
50
40
30
20
10
0
DMSO
50
100
GT (nM)

[Figure 5]

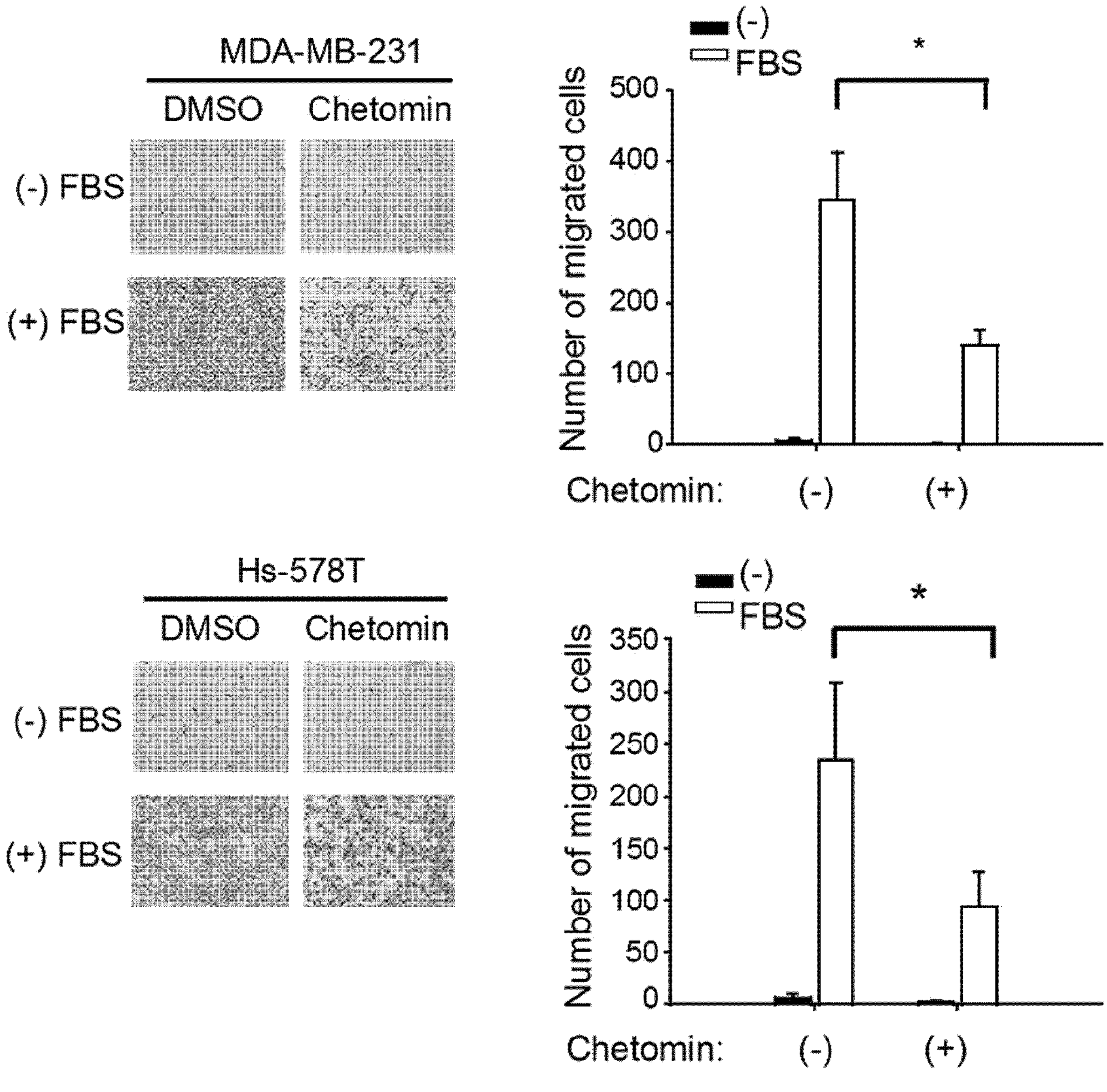
MDA-MB-231
DMSO
Chetomin
(-) FBS
(+) FBS
(-)
FBS
*
Number of migrated cells
500
400
300
200
100
0
Chetomin:
(-)
(+)
Hs-578T
DMSO
Chetomin
(-) FBS
(+) FBS
(-)
FBS
*
Number of migrated cells
350
300
250
200
150
100
50
0
Chetomin:
(-)
(+)

[Figure 6]

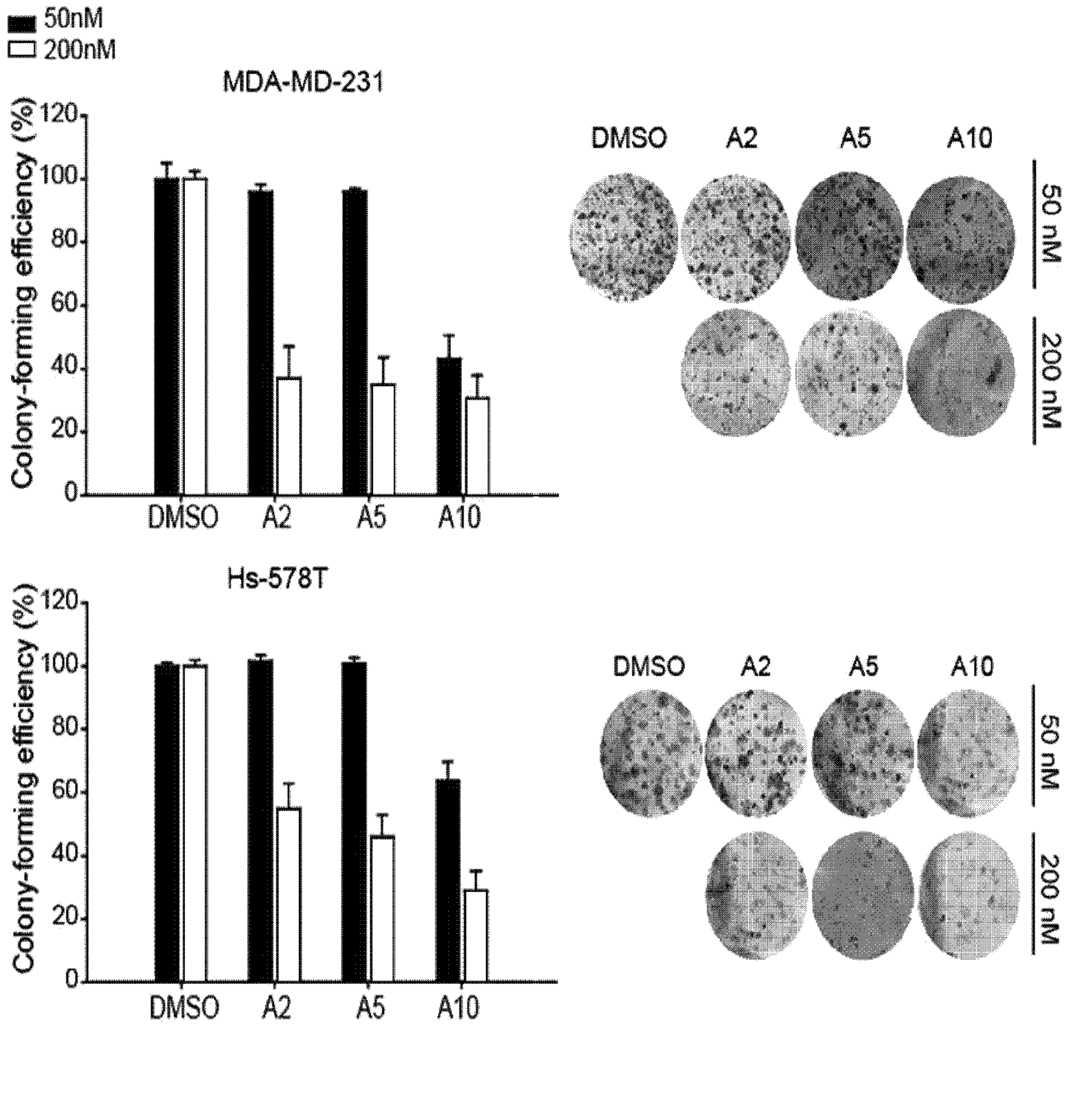
50nM
200nM
MDA-MD-231
Colony-forming efficiency (%)
120
100
80
60
40
20
0
DMSO
A2
A5
A10
DMSO
A2
A5
A10
50 nM
200 nM
Hs-578T
Colony-forming efficiency (%)
120
100
80
60
40
20
0
DMSO
A2
A5
A10
DMSO
A2
A5
A10
50 nM
200 nM

[Figure 7]

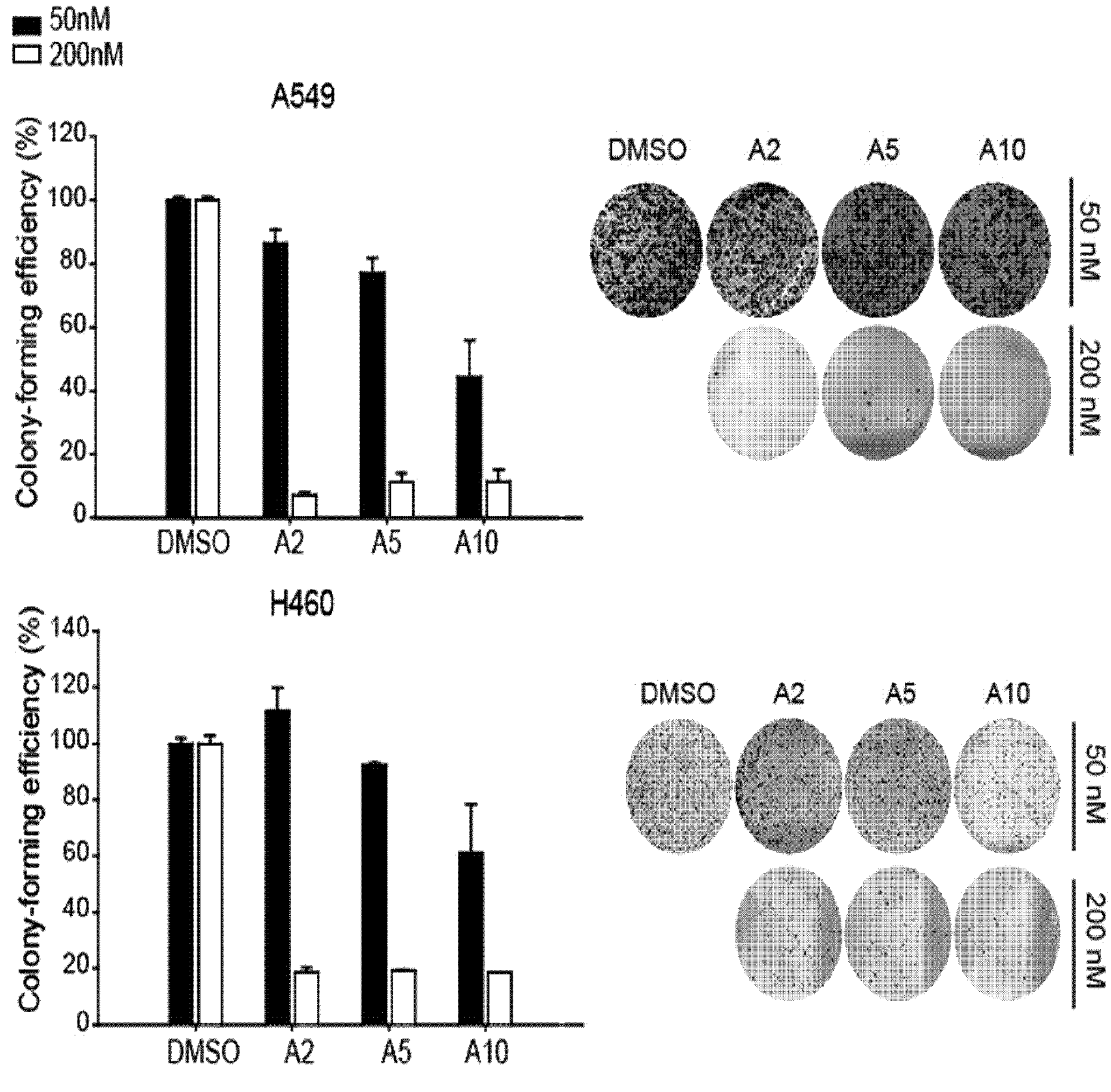
50nM
200nM
A549
Colony-forming efficiency (%)
120
100
80
60
40
20
0
DMSO
A2
A5
A10
DMSO
A2
A5
A10
50 nM
200 nM
H460
Colony-forming efficiency (%)
140
120
100
80
60
40
20
0
DMSO
A2
A5
A10
DMSO
A2
A5
A10
50 nM
200 nM

[Figure 8]

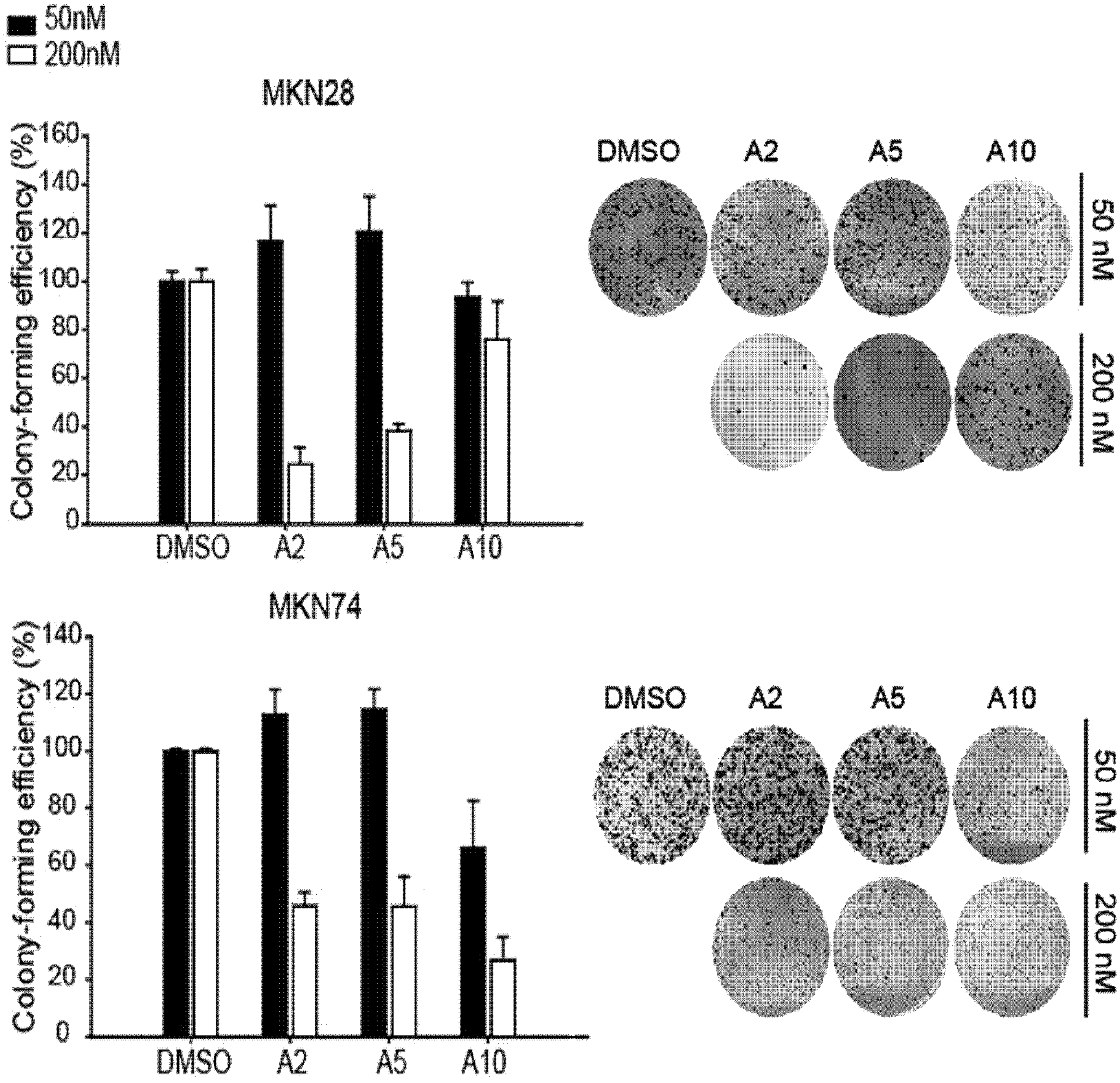
50nM
200nM
MKN28
Colony-forming efficiency (%)
160
140
120
100
80
60
40
20
0
DMSO
A2
A5
A10
DMSO
A2
A5
A10
50 nM
200 nM
MKN74
Colony-forming efficiency (%)
140
120
100
80
60
40
20
0
DMSO
A2
A5
A10
DMSO
A2
A5
A10
50 nM
200 nM

[Figure 9]

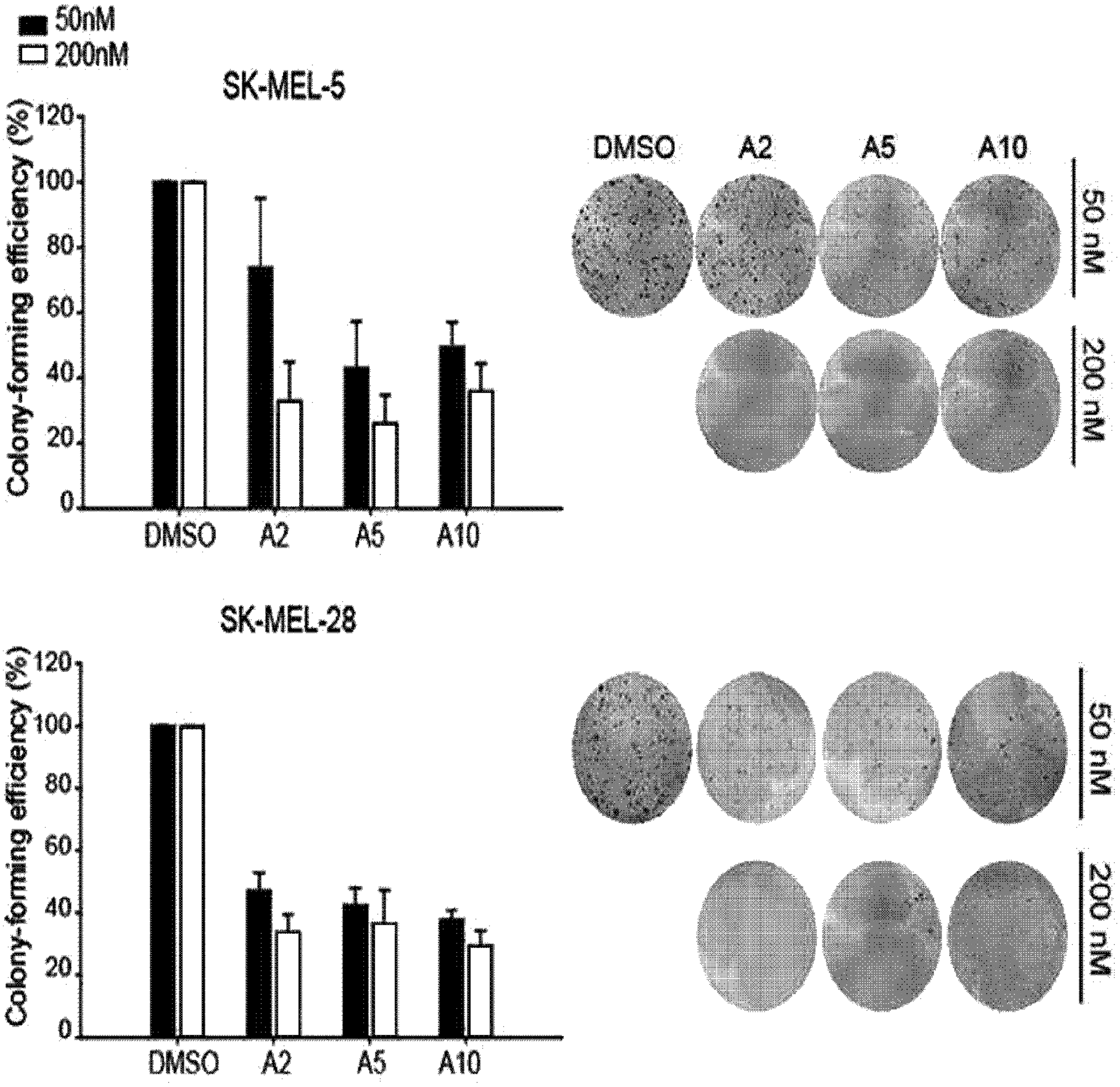
50nM
200nM
SK-MEL-5
Colony-forming efficiency (%)
120
100
80
60
40
20
0
DMSO
A2
A5
A10
DMSO
A2
A5
A10
50 nM
200 nM
SK-MEL-28
Colony-forming efficiency (%)
120
100
80
60
40
20
0
DMSO
A2
A5
A10
50 nM
200 nM

[Figure 10]

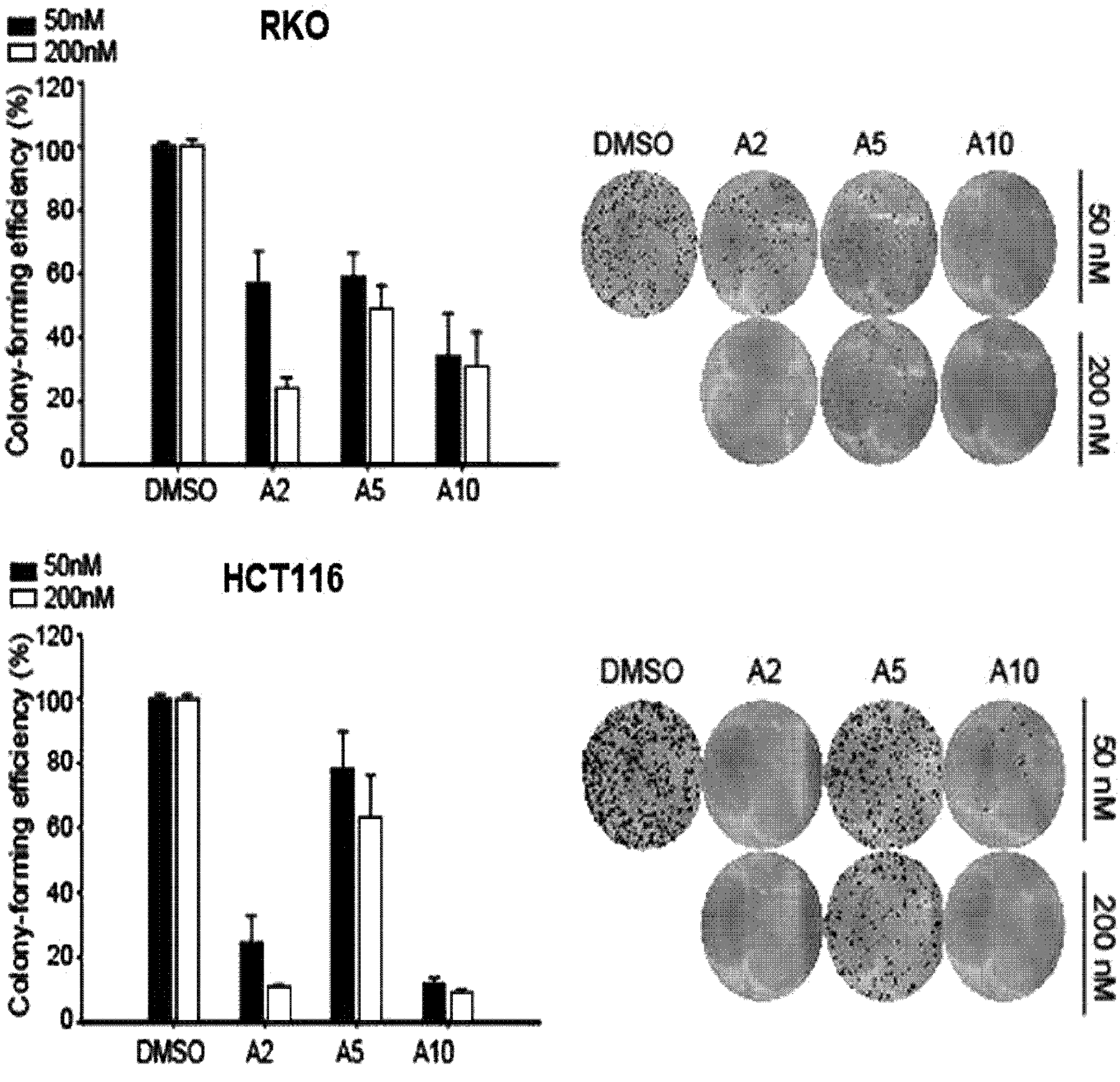
50nM
200nM
RKO
Colony-forming efficiency (%)
120
100
80
60
40
20
0
DMSO
A2
A5
A10
DMSO
A2
A5
A10
50 nM
200 nM
50nM
200nM
HCT116
Colony-forming efficiency (%)
120
100
80
60
40
20
0
DMSO
A2
A5
A10
DMSO
A2
A5
A10
50 nM
200 nM

[Figure 11]

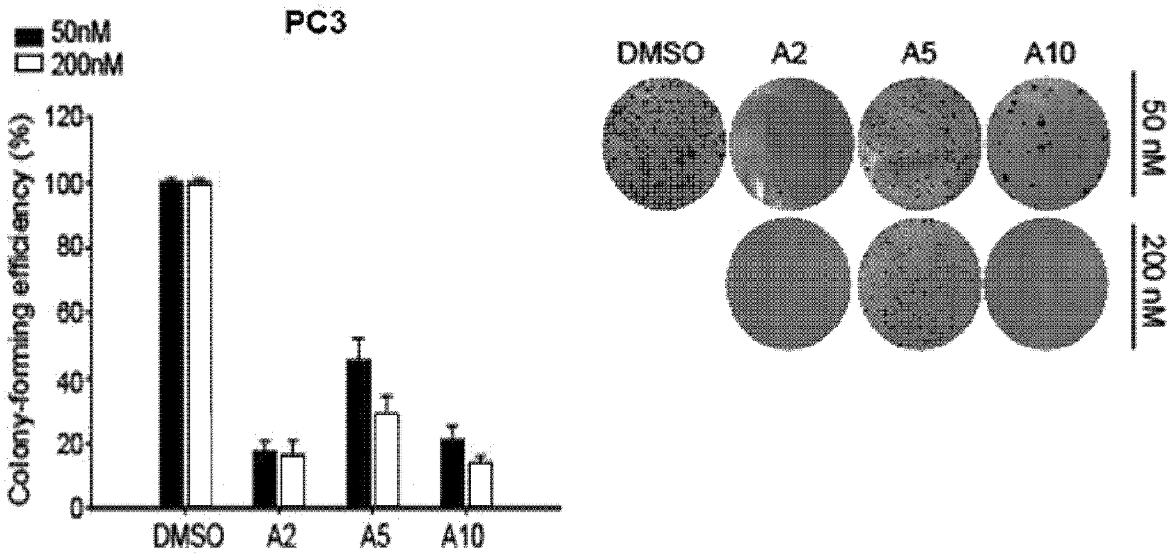


[Figure 12]

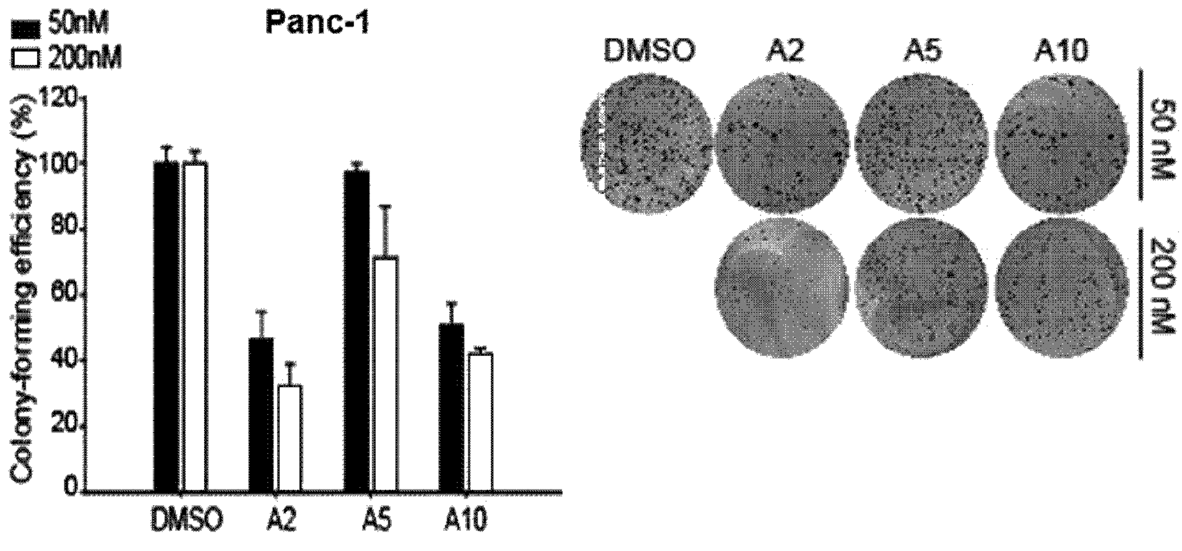

[Figure 13]

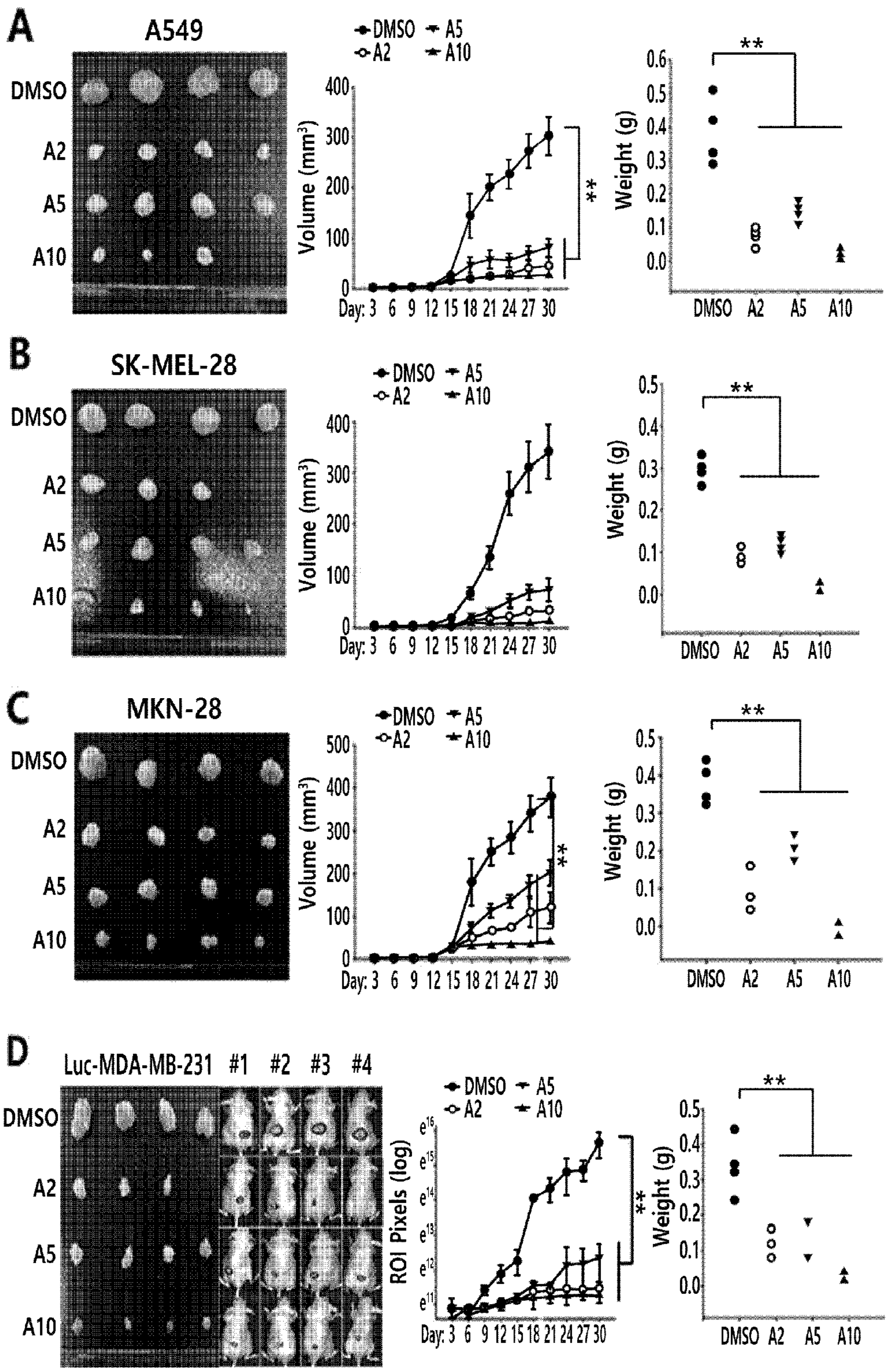
A
A549
DMSO
A2
A5
A10
DMSO
A2
A5
A10
Volume (mm³)
Day: 3 6 9 12 15 18 21 24 27 30
**
Weight (g)
B
SK-MEL-28
C
MKN-28
D
Luc-MDA-MB-231
#1 #2 #3 #4
ROI Pixels (log)

[Figure 14]

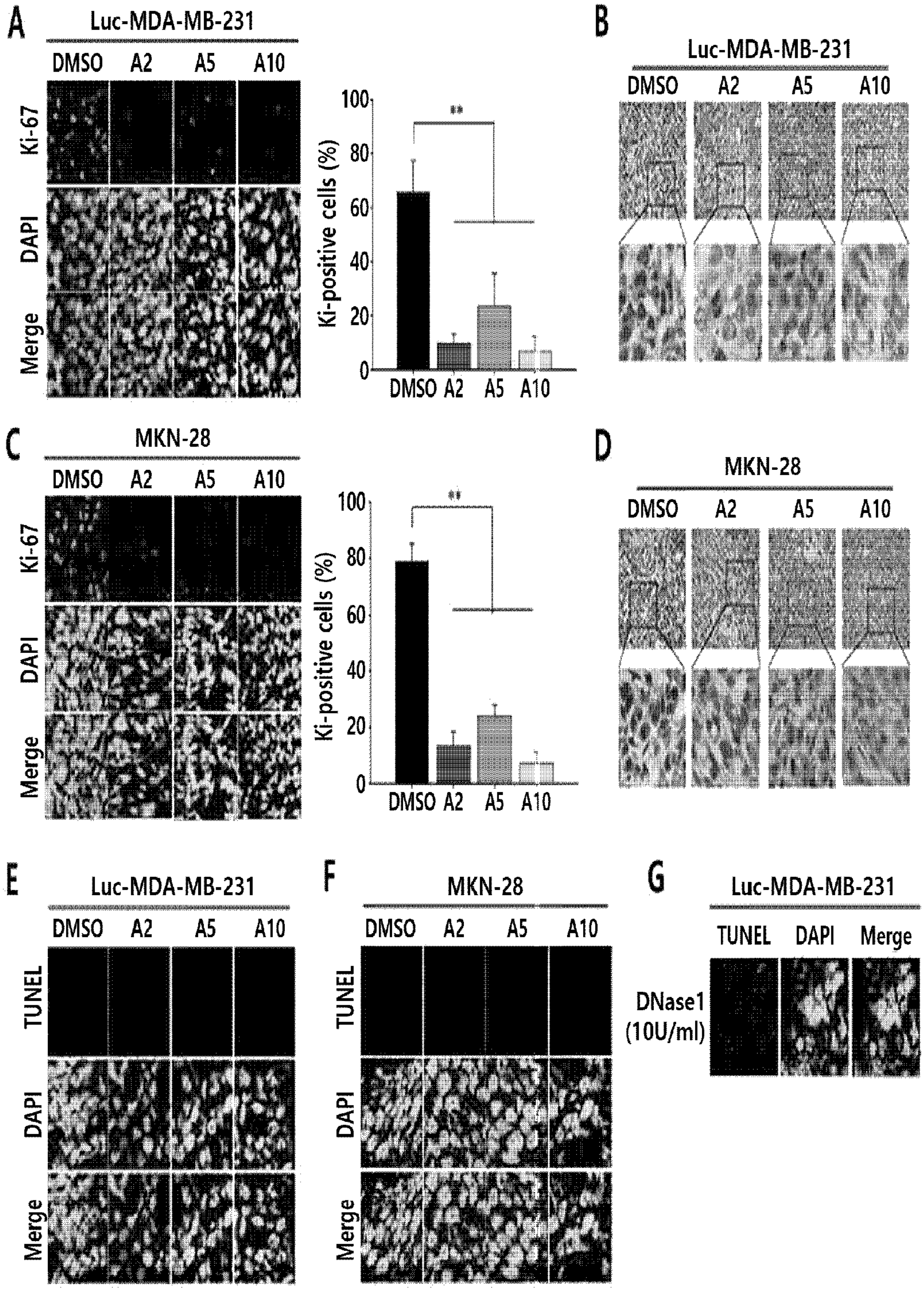
A
Luc-MDA-MB-231
DMSO A2 A5 A10
Ki-67
DAPI
Merge
Ki-positive cells (%)
100
80
60
40
20
0
**
DMSO A2 A5 A10
B
Luc-MDA-MB-231
DMSO A2 A5 A10
C
MKN-28
DMSO A2 A5 A10
Ki-67
DAPI
Merge
Ki-positive cells (%)
**
DMSO A2 A5 A10
D
MKN-28
DMSO A2 A5 A10
E
Luc-MDA-MB-231
DMSO A2 A5 A10
TUNEL
DAPI
Merge
F
MKN-28
DMSO A2 A5 A10
TUNEL
DAPI
Merge
G
Luc-MDA-MB-231
TUNEL DAPI Merge
DNase1
(10U/ml)

[Figure 15]

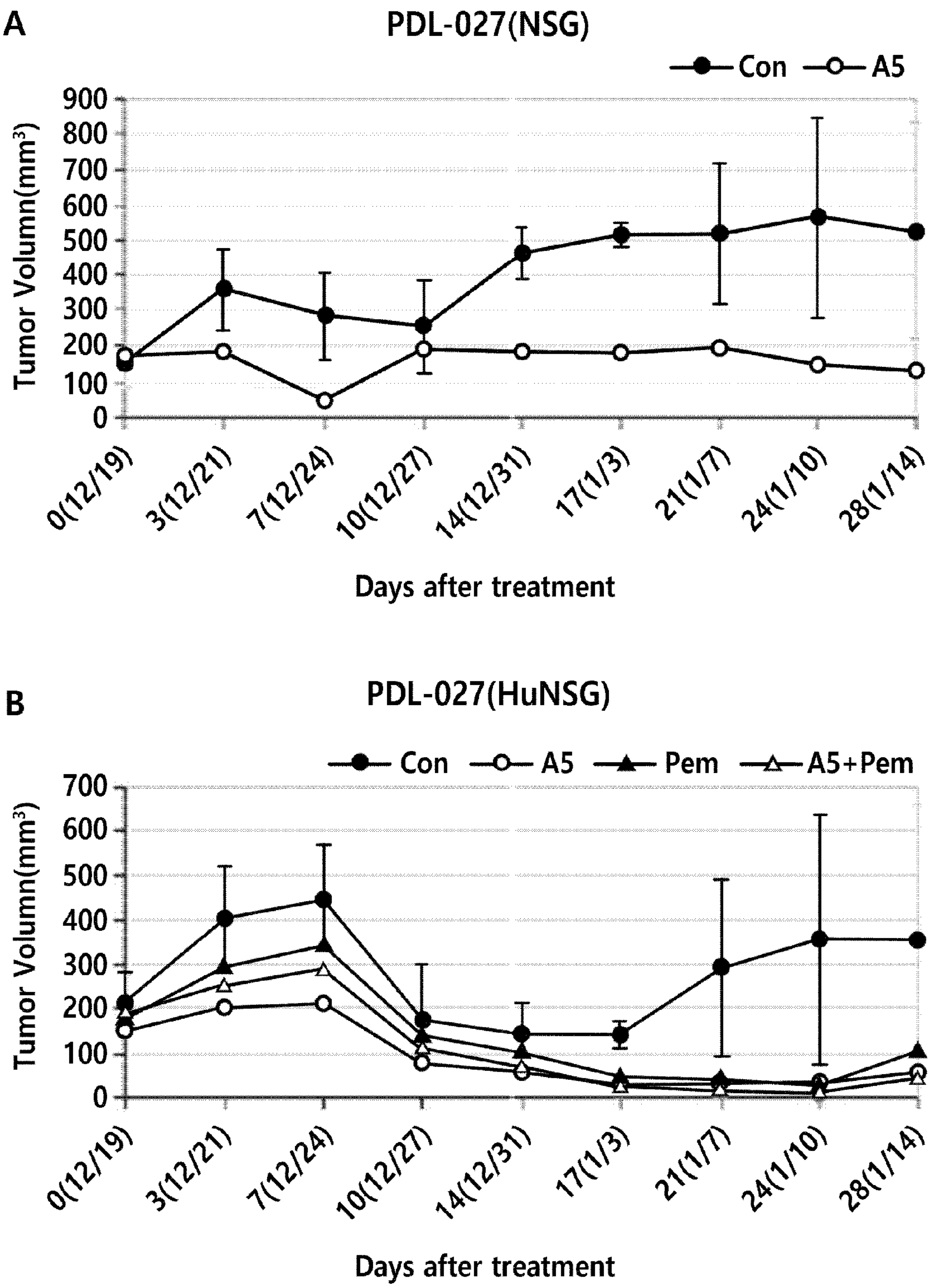
A
PDL-027(NSG)
Con
A5
Tumor Volumn(mm3)
900
800
700
600
500
400
300
200
100
0
0(12/19)
3(12/21)
7(12/24)
10(12/27)
14(12/31)
17(1/3)
21(1/7)
24(1/10)
28(1/14)
Days after treatment
B
PDL-027(HuNSG)
Con
A5
Pem
A5+Pem
Tumor Volumn(mm3)
700
600
500
400
300
200
100
0
0(12/19)
3(12/21)
7(12/24)
10(12/27)
14(12/31)
17(1/3)
21(1/7)
24(1/10)
28(1/14)
Days after treatment

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101633975


### Non-patent literature cited in the description


- **KIRBY** ; **ROBINS**. The Biosynthesis of Mycotoxins,. Academic Press, 1980
- **SHAH** ; **LARSEN**. *Mycopathologia*, 1991, vol. 116, 203-208
- **SEKITA et al.** *Can. J. Microbiol.*, 1981, vol. 27, 766-772
- **BAUMANN et al.** *Chemical Science*, 2015, vol. 6 (8), 4451-4457